# EUROPÄISCHE PATENTSCHRIFT

(11) **EP 0 958 270 B1**
(45) Veröffentlichungstag und Bekanntmachung des Hinweises auf die Patenterteilung: **24.04.2002**
(21) Anmeldenummer: 97953929.3
(22) Anmeldetag: 30.12.1997
(51) Int. Cl.: C07C 45/50, B01J 31/24, B01J 31/26, C07F 9/50, C01G 55/00

(54) **VERFAHREN ZUR HERSTELLUNG VON ALDEHYDEN IN ANWESENHEIT EINER RHODIUM UND SULFONIERTE TRIARYLPHOSPHINE ALS KATALYSATOR ENTHALTENDEN WÄSSRIGEN PHASE**
PROCESS FOR PREPARING ALDEHYDES IN THE PRESENCE OF AN AQUEOUS PHASE CONTAINING RHODIUM AND SULPHONATED TRIARYLPHOSPHINES AS CATALYST
PROCEDE ET PREPARATION D'ALDEHYDES EN PRESENCE D'UNE PHASE AQUEUSE CONTENANT COMME CATALYSEUR DU RHODIUM ET DE LA TRIARYLPHOSPHINE SULFONEE

(30) Priorität: 13.01.1997 DE 19700804
(43) Veröffentlichungstag der Anmeldung: 24.11.1999
(73) Patentinhaber: Celanese Chemicals Europe GmbH, 60439 Frankfurt am Main (DE)
(72) Erfinder: BOGDANOVIC, Sandra, D-60322 Frankfurt am Main (DE); FROHNING, Carl-Dieter, D-46485 Wesel (DE); BAHRMANN, Helmut, D-46499 Hamminkeln (DE)
(86) Internationale Anmeldenummer: EP9707314
(87) Internationale Veröffentlichungsnummer: WO9830526

(56) Entgegenhaltungen:
- WO-A-98/04346
- DE-A- 2 627 354
- DE-A- 3 135 127
- DE-A- 3 412 335
- Patent Abstracts of Japan, Band 96, Nr 2(-); & JP,A,07267890 (KURARAY CO LTD), 1995-10-17

## Beschreibung

Die vorliegende Erfindung betrifft ein Verfahren zur Herstellung von Aldehyden durch Umsetzung von olefinischen Verbindungen mit 6 bis 16 Kohlenstoffatomen mit Wasserstoff und Kohlenmonoxid bei erhöhtem Druck in Anwesenheit einer Rhodium und sulfonierte Triarylphosphine als Katalysator enthaltenden wäßrigen Phase.

Es ist bekannt, durch Umsetzung von Olefinen mit Kohlenmonoxid und Wasserstoff Aldehyde und Alkohole herzustellen. Die Reaktion wird durch Hydridometallcarbonyle, vorzugsweise solcher der Metalle der 8. Gruppe des Periodensystems, katalysiert. Neben Kobalt, das als Katalysatormetall in großem Umfang technische Anwendung findet, hat seit längerer Zeit Rhodium zunehmende Bedeutung erlangt. Im Gegensatz zu Kobalt gestattet Rhodium, die Reaktion bei niedrigem Druck durchzuführen; darüber hinaus werden vorzugsweise geradkettige n-Aldehyde und nur in untergeordnetem Maße iso-Aldehyde gebildet. Schließlich ist auch die Hydrierung der Olefine zu gesättigten Kohlenwasserstoffen bei Anwendung von Rhodium-Katalysatoren deutlich niedriger als bei Anwendung von Kobalt-Katalysatoren.

Bei den in der Technik eingeführten Verfahren wird der Rhodium-Katalysator in Form von modifizierten Hydridorhodiumcarbonylen eingesetzt, die zusätzliche und gegebenenfalls im Überschuß eingesetzte Liganden enthalten. Besonders bewährt haben sich als Liganden tertiäre Phosphine oder Phosphite. ihre Anwendung ermöglicht, den Reaktionsdruck auf Werte unter 30 MPa zu senken.

Probleme wirft bei diesem Verfahren jedoch die Abtrennung der Reaktionsprodukte und die Wiedergewinnung der im Reaktionsprodukt homogenen gelösten Katalysatoren auf. Im allgemeinen destilliert man hierzu das Umsetzungsprodukt aus dem Reaktionsgemisch ab. In der Praxis kann dieser Weg wegen der thermischen Empfindlichkeit der gebildeten Aldehyde und Alkohole aber nur bei der Hydroformylierung niedriger Olefine, d. h. Olefine mit bis zu etwa 5 Kohlenstoffatomen im Molekül; beschritten werden.

Bei der Hydroformylierung langkettiger Olefine oder olefinischer Verbindungen mit funktionellen Gruppen werden Produkte mit hohem Siedepunkt gebildet, die sich destillativ vom homogen gelösten Rhodium-Komplexkatalysator nicht abtrennen lassen. Die thermische Belastung des Destillationsgutes führt durch Dickölbildung zu erheblichen Verlusten an Wertprodukten und an Katalysator durch Zersetzung der Rhodiumkomplexverbindungen.

Die Abtrennung des Katalysators auf thermischem Wege wird durch Anwendung in Wasser löslicher Katalysatorsysteme vermieden. Derartige Katalysatoren sind z. B. in der DE-PS 26 27 354 beschrieben. Die Löslichkeit der Rhodiumkomplexverbindungen wird hierbei durch Verwendung von sulfonierten Triarylphosphinen als Komplexbestandteil erreicht. Die Abtrennung des Katalysators vom Reaktionsprodukt nach Beendigung der Hydroformylierungsreaktion erfolgt bei dieser Verfahrensvariante einfach durch Trennung von wäßriger und organischer Phase, d. h. ohne Destillation und damit ohne zusätzliche thermische Verfahrensschritte. Ein weiteres Merkmal dieser Arbeitsweise ist, daß mit hoher Selektivität aus endständigen Olefinen n-Aldehyde und nur in ganz untergeordnetem Maße iso-Aldehyde gebildet werden. Neben sulfonierten Triarylphosphinen werden als Komplexbestandteile wasserlöslicher Rhodiumkomplexverbindungen auch carboxylierte Triarylphosphine eingesetzt.

Der Einsatz wasserlöslicher Katalysatoren hat sich bei der Hydroformylierung niederer Olefine, insbesondere Ethylen und Propen, bewährt. Setzt man höhere Olefine wie Hexen, Octen oder Decen ein, geht jedoch die Umsetzungsgeschwindigkeit in hohem Maße zurück. Die Wirtschaftlichkeit der Umsetzung in technischem Maßstab ist bei Einsatz von Olefinen mit sechs und mehr Kohlenstoffatomen nicht mehr gegeben.

Um bei der Hydroformylierung höherer Olefine mittels wasserlöslicher Kataiysatoren den Umsatz und/oder die Selektivität der Reaktion zu n-Aldehyden zu steigern, wird der Zusatz eines amphiphilen Reagenz (DE 31 35 127 A1) oder eines Lösungsvermittlers (DE 34 12 335 A1) empfohlen.

Sehr hohe Umsätze werden sowohl gemäß DE 31 35 127 A1 als auch gemäß DE 34 12 335 unter Verwendung quaternärer Ammoniumsalze, die einen langkettigen Alkylrest aufweisen, erhalten, während nichtionische Stoffe auf Basis Polyethylenglykol zu vergleichsweise niedrigen Umsätzen führen.

Wie aus der DE 31 35 127 Tabelle 7 hervorgeht, führt die Hydroformylierung von Dodecen-(1) mittels Rhodium und monosulfoniertem Triphenylpnosphin (3-Ph₂PC₆H₄SO₃Na) ohne Zusatz eines amphiphilen Reagenz zu einem Umsatz von 56 % (Beispiel 77), während der Zusatz von C₁₂H₂₅(OCH₂CH₂)₂₃OH (="Brij 35") zu einer Minderung des Umsatzes auf 37 % (Beispiel 78) führt.

Gemäß DE 34 12 335 (Tabelle 4) führt die Hydroformylierung von Hexen mittels Rhodium und Trinatrium-tri(m-sulfophenyl)phosphin ohne Zusatz eines Lösungsvermittlers zu einem Umsatz von 36 % (Beispiel 10), während ein Zusatz von 2,5 % Triethylenglykol (Beispiel 14) bzw. von 5 % Polyglykol 200 (Beispiel 11) einen Umsatz von 43,5 % bzw. 43 % ergibt. Der Zusatz der Lösungsvermittier hat keine signifikante Steigerung des Umsatzes zur Folge und auch die Erhöhung der Menge von 2,5 % auf 5% Lösungsvermittler bewirkt keine Erhöhung des Umsatzes. Ein sehr hoher Umsatz, nämlich 86 %, wird hingegen mit einem Zusatz von 2,5 % Trimethylhexadecylammoniumbromid erzielt.

Der Einsatz quaternärer Ammoniumsalze als amphiphiles Reagenz oder Lösungsvermittler ist allerdings wegen der schlechten biologischen Abbaubarkeit dieser Verbindungen nicht unproblematisch. So führt die Anwesenheit quaternärer Ammoniumsalze im Abwasser zu Schwierigkeiten bei Abwasseraufarbeitung.

Amphiphile Reagenzien und Lösungsvermittler dienen dazu, den Stofftransport zwischen den einzelnen Phasen und damit die Vermischbarkeit von wäßriger Katalysatorphase und organischer Phase zu begünstigen. Eine Steigerung der Vermischbarkeit von wäßriger Katalysatorphase und organischer Phase geht mit einer erhöhten Löslichkeit von organischer Phase in wäßriger Phase und von wäßriger Phase in organischer Phase einher. Auf diese Weise kann in zunehmendem Maße sowohl amphiphiles Reagenz und Lösungsvermittler als auch Rhodium und wasserlösliches Phosphin in die organische Phase gelangen und nach Phasentrennung mit der organischen Phase ausgetragen werden.

Ferner ist zu erwarten, daß mit steigender Vermischbarkeit von wäßriger Katalysatorphase und organischer Phase die für die Phasentrennung erforderliche Entmischung infolge der Bildung von Emulsionen oder Lösungen nicht mehr oder nicht in genügendem Umfange stattfindet. Eine entsprechende Steigerung der Vermischbarkeit ist insbesondere bei einer Erhöhung des Zusatzes amphiphiler Reagenzien und Lösungsvermittler zu erwarten.

Ein erhöhter Austrag von Rhodium, wasserlöslichem Phosphin und amphiphilem Reagenz oder Lösungsvermittler über die organische Phase ist ebenso wie eine herabgesetzte Entmischbarkeit der Phasen unerwünscht, da Rhodium, wasserlösliches Phosphin und amphiphiles Reagenz oder Lösungsvermittler in der wäßrigen Katalysatorphase verbleiben sollen und eine gute Entmischbarkeit eine unabdingbare Voraussetzung für die erforderliche, die Hydroformylierung abschließende Trennung von organischer und wäßriger Phase ist.

In Hinblick auf die voranstehenden Darlegungen besteht ein Bedarf, ein Verfahren bereitzustellen, das die vorstehend genannten Nachteile vermeidet und sich zudem auf einfache Weise technisch realisieren läßt.

Diese Aufgabe wird gelöst durch ein Verfahren zur Herstellung von Aldehyden, bei dem man eine olefinisch ungesättigte Verbindung mit 6 bis 16 Kohlenstoffatomen mit Wasserstoff und Kohlenmonoxid in Anwesenheit von Lösungsvermittler und einer Rhodium und sulfonierte Triarylphosphine als Katalysator enthaltenden wäßrigen Phase umsetzt, das dadurch gekennzeichnet, ist, daß man in Anwesenheit einer wäßrigen Phase arbeitet, die mehr als 10 Gew.-% und bis zu 70 Gew.-% eines Gemisches von Verbindungen der Formel (1) R(OCH₂CH₂)ₙOR¹ enthält, wobei in der Formel (1) R für Wasserstoff, einen geradkettigen oder verzweigten Alkylrest mit 1 bis 4 Kohlenstoffatomen oder einen Hydroxyalkylrest mit 1 bis 4 Kohlenstoffatomen, R¹ für Wasserstoff oder einen Methylrest und n für eine ganze Zahl von 3 bis 50 steht, daß die Triarylphosphine wenigstens drei -(SO₃)M-Reste enthalten, worin M gleich oder verschieden ist und M für H, ein Alkalimetallion, ein Ammoniumion, ein quaternäres Ammoniumion, ein ½ Erdalkalimetallion oder ½ Zinkion steht und daß die Umsetzung bei 20 bis 170 °C und 1 bis 300 bar stattfindet.

in Hinblick auf die vorstehend geschilderten Befunde der DE 34 12 335 (Tabelle 4, Beispiele 10, 14 und 11), und DE 31 35 127 (Tabelle 7, Beispiele 77 und 78) war es nicht zu erwarten, daß ein Zusatz von Verbindungen der Formel (1) R(OCH₂CH₂)ₙOR¹ in den obenstehend genannten Mengen zu einem signifikanten Anstieg des Umsatzes und gleichzeitig hoher Selektivität bezüglich der Bildung von n-Aldehyden führen würde.

Es ist ferner überraschend, daß der Zusatz vergleichsweise großer bis sehr großer Mengen von Verbindungen der Formel (1) R(OCH₂CH₂)ₙOR¹ nicht einen signifikanten Anstieg von Rhodium und sulfoniertem Triarylphosphin in der organischen Phase und somit einen erhöhten Austrag des Katalysators über die organische Phase bewirkt.

Darüber hinaus war nicht zu erwarten, daß trotz der vergleichsweise großen bis sehr großen Mengen von Verbindungen der Formel (1), die Entmischbarkeit von organischer Phase und wäßriger Katalysatorphase so hoch ist, daß eine schnelle Trennung von organischer Phase und wäßriger Katalysatorphase gewährleistet ist. Überraschenderweise werden schwertrennbare Emulsionen oder homogene, nicht trennbare Phasen respektive Lösungen nicht gebildet.

Die den Katalysator und die Verbindung der Formel (1) R(OCH₂CH₂)ₙOR¹ enthaltende wäßrige Phase läßt sich auf vergleichsweise einfache Art herstellen, indem man ein wasserlösliches Rhodiumsalz, die sulfonierten Triarylphosphine und die Verbindung der Formel (1) in Wasser löst.
Geeignete Rhodiumsalze sind, ohne Anspruch auf Vollständigkeit zu erheben: Rhodium-III-sulfat, Rhodium-III-nitrat, Rhodium-III-carboxylate wie Rhodiumacetat, Rhodiumpropionat, Rhodiumbutyrat, Rhodium-2-ethylhexanoat.

Man kann die wäßrige Phase unmittelbar in die Hydroformylierung einsetzen oder sie zuvor einer Präformierung des Katalysators unter Reaktionsbedingungen unterwerfen, um sie anschließend in präformierter Form zu verwenden.

Als olefinische Verbindung kann man ein aliphatisches Olefin, cycloaliphatisches Olefin oder araliphatisches Olefin mit 6 bis 16, insbesondere 6 bis 10 Kohlenstoffatomen, vorzugsweise ein aliphatisches α-Olefin oder cycloaliphatisches Olefin mit 6 bis 12, insbesondere 6 bis 10 Kohlenstoffatomen einsetzen.

Die olefinische Verbindung kann eine oder mehr als eine Kohlenstoff-Kohlenstoff-Doppelbindung enthalten. Die Kohlenstoff-Kohlenstoff-Doppelbindung kann endständig oder innenständig angeordnet sein. Bevorzugt sind olefinische Verbindungen mit endständiger Kohlenstoff-Kohlenstoff-Doppelbindung.

Beispiele für α-olefinische Verbindungen (mit endständiger Kohlenstoff-Kohlenstoff-Doppelbindung) sind Alkene, Alkylalkenoate, Alkylenalkanoate, Alkenylalkylether und Alkenole.

Ohne Anspruch auf Vollständigkeit seien als α-olefinische Verbindungen 1-Hexen, 1-Hepten, 1-Octen, 1-Decen, 1-Dodecen, 1-Hexadecen, 2-Ethyl-1-hexen, Styrol, 3-Phenyl-1-propen, 1,4-Hexadien, 1,7-Octadien, 3-Cyclohexyl-1-buten, Hex-1-en-4-ol, Oct-1-en-4-ol, 3-Butenylacetat, Allylpropionat, Allylbutyrat, 4-Vinylcyclohexen, n-Propyl-7-octenoat, 7-Octensäure, 5-Hexenamid, 1-Methoxy-2,7-octadien, 3-Methoxy-1,7-octadien genannt.

Als Beispiele weiterer geeigneter olefinischer Verbindungen seien Diisobutylen, Tripropylen, Octol oder Dimersol (Dimerisierungsprodukte von Butenen), Tetrapropylen, Cyclohexen, Dicyclopentadien, acyclische, cyclische oder bicyclische Terpene, wie Myrcen, Limonen und Pinen erwähnt.

Unter sulfonierten Triarylphosphinen werden Phosphine mit einem oder zwei Phosphoratomen verstanden, die je Phosphoratom drei Arylreste besitzen, die Arylreste gleich oder verschieden sind und für einen Phenyl-, Naphthyl-, Biphenyl-, Phenylnaphthyl- oder Binaphthyl-Rest, insbesondere Phenyl-, Biphenyl oder Binaphthyl-Rest stehen, die Arylreste mit dem Phosphoratom direkt oder über eine -(CH₂)ₓ-Gruppe, worin x für eine ganze Zahl von 1 bis 4, insbesondere 1 bis 2, bevorzugt 1 steht, verbunden sind, und wenigstens drei -(SO₃)M Reste enthalten, worin M gleich oder verschieden ist und für H, ein Alkalimetallion. ein Ammoniumion, ein quaternäres Ammoniumion, ein ½ Erdalkalimetallion oder ½ Zinkion, insbesondere ein Alkalimetallion, ein Ammoniumion oder quaternäres Ammoniumion, bevorzugt ein Alkalimetallion steht. Die -SO₃M Reste sitzen üblicherweise als Substituenten an den Arylresten und verleihen den Triarylphosphinen die erforderliche Wasserlöslichkeit.

Man verwendet als sulfonierte Triarylphosphine Verbindungen der Formel (2) 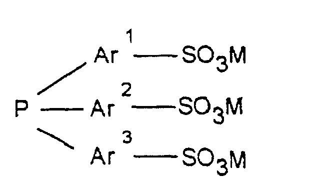 worin Ar¹, Ar² und Ar³ gleich oder verschieden sind und jeweils einen Phenyl- oder Naphthylrest, insbesondere einen Phenylrest, bedeuten und M gleich oder verschieden, insbesondere gleich sind und für ein Alkalimetallion, ein Ammoniumion, ein quaternäres Ammoniumion oder ein 1/2 Erdkakalimetallion oder 1/2 Zinkion, insbesondere ein Akalimetallion oder Ammoniumion. bevorzugt ein Alkalimetallion, besonders bevorzugt ein Natriumion, steht.

Besonders geeignet ist Trinatrium-tri-(m-sulfophenyl)phosphin als sulfoniertes Triarylphosphin. Dieses Trinatriumsalz des Tri-(meta-sulfophenyl)phosphins enthält aufgrund seiner Herstellung durch Sulfonierung von Triphenylphosphin auch noch Anteile an mono- und disulfonierten Verbindungen und geringe Anteile der entsprechenden Phosphinoxide.

Das Trinatrium-tri(m-sulfophenyl)phosphin entspricht der nachfolgenden Formel:

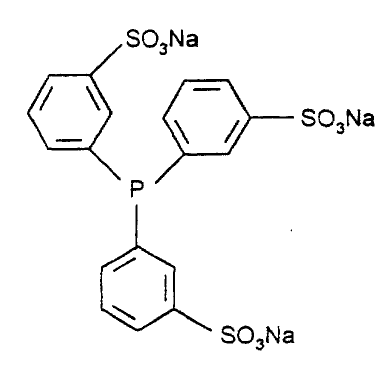

Die sulfonierten Triarylphosphine mit zwei Phosphoratomen können beispielsweise einen Rest -(CH₂)ₓ-Ar-Ar-(CH₂)ₓ- enthalten, worin x für eine ganze Zahl von 1 bis 4, insbesondere 1 bis 2, vorzugweise 1 steht, Ar-Ar Biphenyl oder Binaphthyl bedeutet, die -(CH₂)ₓ-Gruppe mit der einen Bindung jeweils in ortho-Stellung zu der die beiden Arylreste verbindenden Aryl-Arylbindung Ar-Ar steht und mit der anderen Bindung jeweils mit einem Phosphoratom, das jeweils zwei weitere, gleiche oder verschiedene Arylreste, insbesondere Phenylreste besitzt, verbunden ist. Diese zwei Phosphoratome enthaltenden Triarylphosphine weisen wenigstens drei -SO₃M Reste, insbesondere vier bis acht -SO₃M Reste, auf, worin M die vorstehend genannte Bedeutung besitzt. Die -SO₃M Reste sitzen üblicherweise an den Arylresten des Restes -(CH₂)ₓ-Ar-Ar-(CH₂)ₓ- und an den zwei weiteren Arylresten, die mit dem Phosphor verbunden sind.

Beispiele für derartige zwei Phosphoratome enthaltende sulfonierte Triarylphosphine sind, ohne Anspruch auf Vollständigkeit zu erheben, durch die nachfolgenden Formeln (3) und (4) gegeben:

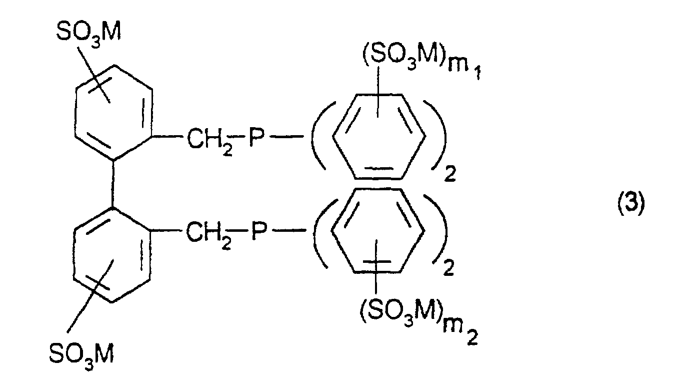

In (3) steht ein jedes m₁ und m₂ unabhängig voneinander für 0 oder 1, wobei die Verbindung der Formel (3) drei bis sechs -SO₃M Gruppen enthält.

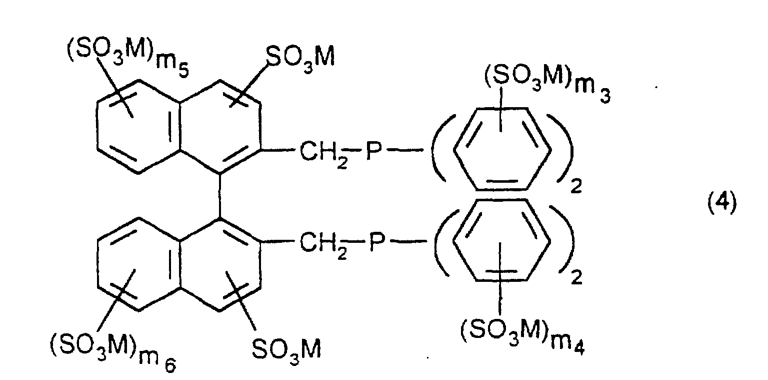

In (4) steht ein jedes m₃, m₄, m₅ und m₆ unabhängig voneinander für 0 oder 1, wobei die Verbindung der Formel (4) vier bis acht -SO₃M Gruppen enthält.

Aufgrund der Herstellung durch Sulfonierung der entsprechenden Phosphine der Formel (3a) und (4a), die keine -SO₃M Gruppen enthalten, 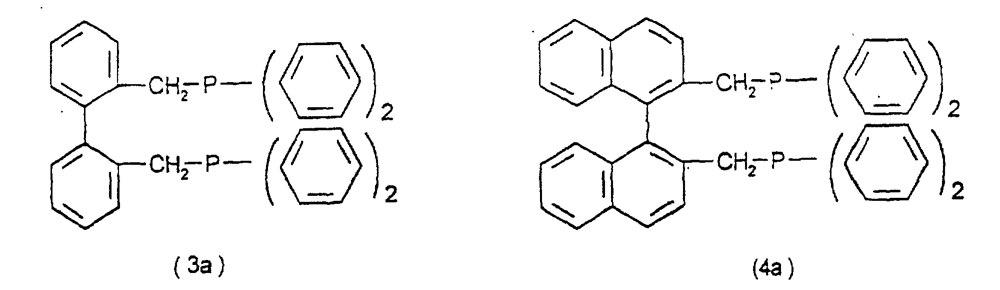 erhält man üblicherweise Gemische von Verbindungen (3) und (4) mit unterschiedlicher Anzahl von -SO₃M Gruppen. So enthält eine Verbindung der Formel (3) oder (4), die beispielsweise drei SO₃M Gruppen enthält, auch Verbindungen mit lediglich zwei -SO₃M Gruppen aber auch Verbindungen mit vier oder fünf -SO₃M Gruppen. Eine Verbindung der Formel (3) oder (4) mit beispielsweise fünf -SO₃M Gruppen enthält üblicherweise auch Verbindungen mit lediglich drei oder vier -SO₃M Gruppen aber auch Verbindungen mit sechs oder sieben -SO₃M Gruppen.

Verbindungen der Formel (3) besitzen maximal sechs -SO₃M Gruppen, während Verbindungen der Formel (4) maximal acht -SO₃M Gruppen aufweisen.

Aus diesem Grund gelangen in der Regel Mischungen von Verbindungen der Verbindungen (3) und (4) mit unterschiedlicher Anzahl von -SO₃M Gruppen zum Einsatz.

Die vorstehend beschriebenen sulfonierten Triarylphosphine besitzen aufgrund ihrer Sulfonat-Reste eine für die Durchführung des Verfahrens ausreichende Löslichkeit in Wasser.

Man setzt die Rhodium und die Verbindungen der Formel (2) als Katalysator und die Verbindung der Formel (1) enthaltende wäßrige Phase üblicherweise entsprechend einer Menge von 2 x 10⁻⁶ bis 5 x 10⁻², insbesondere 5 x 10⁻⁵ bis 5 x 10⁻², bevorzugt 1 x 10⁻⁴ bis 1 x 10⁻³ Mol Rhodium pro Mol olefinischer Verbindung ein.

Die Rhodiummenge hängt auch von der Art der zu hydroformylierenden olefinischen Verbindung ab. Obgleich niedrigere Katalysatorkonzentrationen möglich sind, können sie sich im Einzelfall als nicht besonders zweckmäßig erweisen, da die Reaktionsgeschwindigkeit zu gering und daher nicht wirtschaftlich genug sein kann. Die obere Katalysatorkonzentration kann bis 1 x 10⁻¹ Mol Rhodium pro Mol olefinische Verbindung betragen. Durch vergleichsweise hohe Rhodiumkonzentrationen ergeben sich allerdings keine besonderen Vorteile.

Man setzt die Rhodium und das trisulfonierte Triarylphosphin der Formel (2) als Katalysator und die Verbindung der Formel (1) R(OCH₂CH₂)ₙOH enthaltende wäßrige Phase und die olefinische Verbindung üblicherweise im Volumenverhältnis 10 : 1 bis 1 : 10, insbesondere 5 : 1 bis 1 : 5, bevorzugt 2 : 1 bis 1 : 2 ein.

Man setzt Rhodium und sulfonierte Triarylphosphine im Molverhältnis 1 : 5 bis 1 : 2000.

Verwendet man ein sulfoniertes Triarylphosphin mit einem Phosphoratom, beispielsweise eine Verbindung der Formel (2), so setzt man üblicherweise Rhodium und sulfoniertes Triarylphosphan im Molverhältnis 1 : 10 bis 1 : 1000, insbesondere 1 : 50 bis 1 : 200, bevorzugt 1 : 80 bis 1 : 120, ein.

Verwendet man ein sulfoniertes Triarylphosphin mit zwei Phosphoratomen, beispielsweise Verbindungen der Formel (3) und (4), so setzt man Rhodium und sulfoniertes Triarylphosphin üblicherweise im Molverhältnis 1 : 5 bis 1 : 100, insbesondere 1 : 5 bis 1 : 50, bevorzugt 1 : 8 bis 1 : 15 ein.

Die wäßrige Phase enthält 20 bis 2000 ppm Rhodium. Falls man sulfoniertes Triarylphosphin mit einem Phosphoratom, beispielsweise Verbindungen der Formel (2), einsetzt, so verwendet man in den meisten Fällen eine wäßrige Phase, die 100 bis 1000, insbesondere 200 bis 500, bevorzugt 300 bis 400 ppm Rhodium enthält.

Falls man ein sulfoniertes Triarylphosphin mit zwei Phosphoratomen, beispielsweise Verbindungen der Formel (3) und/oder (4) einsetzt, so verwendet man in den meisten Fällen eine wäßrige Phase, die 20 bis 500, insbesondere 30 bis 150, bevorzugt 40 bis 100 ppm Rhodium enthält.

Die Art der umzusetzenden olefinischen Verbindung kann in gewissem Umfange auch die Menge der einzusetzenden Verbindung der Formel (1) R(OCH₂CH₂)ₙOR¹ beeinflussen.

Setzt man als olefinische Verbindung ein aliphatisches α-Olefin oder ein cycloaliphatisches Olefin mit jeweils 6 bis 8 Kohlenstoffatomen ein, so hat es sich häufig als sinnvoll erwiesen, die Umsetzung in Anwesenheit einer wäßrigen Phase durchzuführen, die mehr als 10 Gew.-% und bis zu 50 Gew.-%, insbesondere 20 bis 40 Gew.-% der Verbindung der Formel (1) enthält.

Setzt man als olefinische Verbindung ein aliphatisches α-Olefin oder ein cycloaliphatisches Olefin mit jeweils 9 bis 12 Kohlenstoffatomen ein, so hat es sich häufig als sinnvoll erwiesen, die Umsetzung in Anwesenheit einer 30 bis 70, insbesondere 50 bis 60 Gew.-% der Verbindung der Formel (1) enthaltenden wäßrigen Phase durchzuführen.

An dieser Stelle sei der Vollständigkeit halber erwähnt, daß es sich bei den Verbindungen der Formel (1) R(OCH₂CH₂)ₙOR¹, worin R für Wasserstoff, einen geradkettigen oder verzweigten Alkylrest mit 1 bis 4 Kohlenstoffatomen oder einen Hydroxyalkylrest mit 1 bis 4 Kohlenstoffatomen, insbesondere für Wasserstoff, einen Alkylrest mit 1 bis 2 Kohlenstoffatomen oder einen Hydroxyalkylrest mit 1 bis 3 Kohlenstoffatomen, bevorzugt für Wasserstoff, Methyl, Hydroxymethyl oder Hydroxypropyl und R¹ für Wasserstoff oder einen Methylrest, insbesondere Wasserstoff steht, um Stoffe handelt, die sich in Wasser in ausreichendem Umfange lösen.

Es sei an dieser Stelle auf die nachfolgenden Verbindungen der Formel (1), worin R¹ für Wasserstoff steht, hingewiesen, die von besonderem Interesse sind.

Ohne Anspruch auf Vollständigkeit zu erheben, seien als Verbindungen der Formel R(OCH₂CH₂)ₙOH, Polyethylenglykol der Formel H(OCH₂CH₂)ₙOH mit einem mittleren Molgewicht von ungefähr 200 (PEG 200), 400 (PEG 400), 600 (PEG 600) oder 1000 (PEG 1000), Verbindungen der Formel CH₃(OCH₂CH₂)ₙOH mit einem mittleren Molgewicht von ungefähr 350 (M 350), 500 (M 500) oder 750 (M 750) oder Verbindungen der Formel CH₃CHOHCH₂(OCH₂CH₂)ₙOH mit einem mittleren Molgewicht von ungefähr 300 (300 PR), 450 (450 PR), 600 (600 PR) oder 1000 (1000PR), insbesondere Polyethylenglykol mit einem mittleren Molgewicht von ungefähr 400 (PEG 400), und 600 (PEG 600), eine Verbindung der Formel CH₃(OCH₂CH₂)ₙOH mit einem mittleren Molgewicht von 500 (M 500) oder eine Verbindung der Formel CH₃CHOHCH₂(OCH₂CH₂)ₙOH mit einem mittleren Molgewicht von 450 (450 PR) und 600 (600 PR) genannt.

Es ist unter PEG 200 ein Gemisch von Polyethylenglykolen der Formel H(OCH₂CH₂)ₙOH, worin n für eine ganze Zahl von 3 bis 6 steht, unter PEG 400 ein Gemisch von Polyethylenglykolen der Formel H(OCH₂CH₂)ₙOH, worin n für eine ganze Zahl von 7 bis 10 steht, unter PEG 600 ein Gemisch von Polyethylenglykolen der Formel H(OCH₂CH₂)ₙOH, worin n für eine ganze Zahl von 11 bis 16 steht und unter PEG 1000 ein Gemisch von Polyethylenglykolen der Formel H(OCH₂CH₂)ₙOH, worin n für eine ganze Zahl von 15 bis 30 steht, zu verstehen. Diesen Gemischen ist jeweils ein entsprechendes mittleres Molgewicht von etwa 200 (PEG 200), etwa 400 (PEG 400), etwa 600 (PEG 600) respektive etwa 1000 zuzuordnen.

Es ist unter M 350 ein Gemisch von Verbindungen der Formel CH₃(OCH₂CH₂)ₙOH, worin n für eine ganze Zahl von 5 bis 9 steht, unter M 500 ein Gemisch von Verbindungen der Formel CH₃(OCH₂CH₂)ₙOH, worin n für eine ganze Zahl von 9 bis 13 steht, unter M 750 ein Gemisch von Verbindungen der Formel CH₃(OCH₂CH₂)ₙOH, worin n für eine ganze Zahl von 12 bis 20 steht, zu verstehen. Diesen Gemischen ist jeweils ein entsprechendes mittleres Molgewicht von etwa 350 (M 350), etwa 500 (M 500) respektive 750 (M 750) zuzuordnen.

Es ist unter 300 PR ein Gemisch von Verbindungen der Formel R(OCH₂CH₂)ₙOH, worin R für einen β-Hydroxypropylrest CH₃CHOHCH₂- und n für eine ganze Zahl von 6 bis 9 steht, unter 450 PR ein Gemisch von Verbindungen der Formel R(OCH₂CH₂)ₙOH, worin R für einen β-Hydroxypropylrest CH₃CHOHCH₂- und n für eine ganze Zahl von 8 bis 14 steht, unter 600 PR ein Gemisch von Verbindungen der Formel R(OCH₂CH₂)ₙOH, worin R für einen β-Hydroxypropylrest CH₃CHOHCH₂- und n für eine ganze Zahl von 12 bis 20 steht, unter 1000 PR ein Gemisch von Verbindungen der Formel R(OCH₂CH₂)ₙOH, worin R für einen β-Hydroxypropylrest CH₃CHOHCH₂- und n für eine ganze Zahl von 18 bis 26 steht, zu verstehen. Diesen Gemischen ist jeweils ein entsprechendes mittleres Molgewicht von etwa 300 (300 PR), etwa 450 (450 PR), etwa 600 (600 PR) respektive etwa 1000 (1000 PR) zuzuordnen.

In einer Reihe von Fällen hat es sich bewährt, ein Polyethylenglykol der Formel H(OCH₂CH₂)ₙOH, worin n für eine ganze Zahl von 3 bis 50, insbesondere 4 bis 30, bevorzugt 5 bis 20, besonders bevorzugt 6 bis 12, steht, als Verbindung der Formel (1) einzusetzen.

Es hat sich auch bewährt, eine Verbindung (Halbether) der Formel R(OCH₂CH₂)ₙOH, worin R für einen Methylrest oder β-Hydroxypropylrest und n für eine ganze Zahl von 3 bis 50, insbesondere 4 bis 30, bevorzugt 5 bis 20 steht, als Verbindung der Formel (1) einzusetzen.

Es lassen sich auch beliebige Mischungen der Verbindungen der Formel (1), nämlich Polyethylenglykole, Polyethylenglykolether (Halbether) und Polyethylenglykoldiether einsetzen.

Man führt die Umsetzung in Gegenwart von Wasserstoff und Kohlenmonoxid durch. Das Molverhältnis von Wasserstoff zu Kohlenmonoxid kann in weiten Grenzen gewählt werden und liegt üblicherweise bei 1 : 10 bis 10 : 1, insbesondere 5 : 1 bis 1 : 5, bevorzugt 2 : 1 bis 1 : 2, besonders bevorzugt 1,2 : 1 bis 1 : 1,2. Besonders einfach gestaltet sich das Verfahren, wenn man Wasserstoff und Kohlenmonoxid im Molverhältnis 1 : 1 oder annähernd 1 : 1 einsetzt.

Für viele Fälle reicht es aus, die Umsetzung bei einer Temperatur von 50 bis 150, inbesondere 100 bis 140 °C durchzuführen.

In einer Vielzahl von Fällen hat es sich als nützlich erwiesen, die Umsetzung bei einem Druck von 10 bis 200, insbesondere 20 bis 150, bevorzugt 30 bis 80 bar durchzuführen.

Während der Umsetzung ist sicherzustellen, daß für eine gute Durchmischung von organischer Phase, wäßriger Phase und Kohlenmonoxid/Wasserstoff gesorgt wird. Dies kann beispielsweise durch intensives Rühren und/oder Umpumpen von organischer und wäßriger Phase bewirkt werden. In der organischen Phase befinden sich üblicherweise die olefinische Verbindung, die erzeugten Aldehyde sowie geringe Anteile der wäßrigen Phase, während die wäßrige Phase üblicherweise Rhodium, die sulfonierten Triarylphosphine, die Verbindung der Formel (1), Wasser und geringe Anteile der organischen Phase enthält.

Es sei an dieser Stelle nochmals darauf hingewiesen, daß die Reaktionsbedingungen, insbesondere Rhodiumkonzentration, Druck und Temperatur auch von der Art der zu hydroformylierenden olefinischen Verbindung abhängen. Vergleichsweise reaktive olefinische Verbindungen erfordern geringe Rhodiumkonzentrationen, niedrige Drücke und niedrige Temperaturen. Hingegen benötigt die Umsetzung relativ reaktionsträger olefinischer Verbindungen größere Rhodiumkonzentrationen, höhere Drücke und höhere Temperaturen.

Das Verfahren läßt sich mit besonders gutem Erfolg ausführen, wenn man eine α-olefinische Verbindung einsetzt. Es lassen sich jedoch auch andere olefinische Verbindungen mit innenständigen Kohlenstoff-Kohlenstoff-Doppelbindungen mit guten Ergebnissen umsetzen.

Das Hydroformylierungsgemisch wird nach Abschluß der Umsetzung durch Druckentspannung von Kohlenmonoxid und Wasserstoff befreit und das Reaktionsprodukt, gegebenenfalls nach Abkühlen, von der den Katalysator und die Verbindung der Formel (1) enthaltenden wäßrigen Phase durch Phasentrennung abgetrennt.

Die den Katalysator und die Verbindung der Formel (1) enthaltende wäßrige Phase kann wieder in das erfindungsgemäße Verfahren zurückgeführt werden, während die abgetrennte, das Reaktionsprodukt enthaltende organische Phase, beispielsweise durch fraktionierte Destillation, aufgearbeitet wird.

Das Verfahren läßt sich kontinuierlich und diskontinuierlich durchführen.

Die nachfolgenden Beispiele beschreiben die Erfindung näher, ohne sie zu beschränken.

### Experimenteller Teil:

### 1. Hydroformylierung von 1-Hexen

### Beispiel 1a) (Vergleichsversuch zu Beispiel 1b) ohne Zusatz von Polyethylenglykol)

### I Herstellung der Katalysatorphase und Präformierung

60 mg (0,233 mmol) Rhodium(III)acetat werden in 39 ml einer 0,6 M wäßrigen Lösung von Trinatrium-tri(m-sulfophenyl)phosphin (Na-TPPTS) entsprechend einem Molverhältnis Rhodium zu Ligand von 1 : 100 und 21 ml entgastem destillierten Wasser gelöst und in einen 200 ml Stahlautoklav unter Stickstoffstrom gegeben. Diese Katalysatorlösung wird unter Rühren bei 25 bar Synthesegasdruck (CO/H₂ = 1/1) während 3 Stunden auf 125 °C erwärmt, wobei die Lösung eine gelbe Farbe annimmt.

### II Hydroformylierung

Zu der präformierten Katalysatorlösung aus I werden bei einem Reaktionsdruck von 30 bar und bei 125 °C 30 ml (240 mmol) 1-Hexen über einen vorgeschalteten 200 ml Stahlautoklav mit leichtem Überdruck zugegeben. Das Verhältnis von Olefin zu Rhodium beträgt 1039 : 1. Die Hydroformylierungsreaktion wird durch Einschalten des Magnetrührers in Gang gesetzt. Während einer Reaktionszeit von 3 Stunden wird die Temperatur auf 125 °C gehalten und der Reaktionsdruck in einem Druckband von +/- 2 bar durch manuelles Zudosieren von Synthesegas konstant gehalten. Nach Ablauf von 3 Stunden werden Rührer und Heizung abgeschaltet, der Autoklav wird auf 40 bis 50 °C abgekühlt und die obere Produktphase von der Katalysatorphase in einem Scheidetrichter getrennt. Produktphase und Katalysatorphase werden gewogen. Die Zusammensetzung der Produktphase wird mittels Gaschromatographie und ¹H-NMR-Spektroskopie ermittelt und aus der Zusammensetzung die Ausbeute an Hydroformylierungsprodukten und das Verhältnis von n- zu iso-Heptanal bestimmt. Der Rhodiumgehalt der organischen Phase wird elementaranalytisch mittels Graphitrohr-Atomabsorptionsspektrometrie nach Probenaufschluß bestimmt. Die Ausbeute an Hydroformylierungsprodukten beträgt 31,2 %, das n/iso-Verhältnis beträgt 98 : 2. Die organische Phase enthält < 0,05 ppm Rh. (Beispiel 1a) in Tabelle 1)

### Beispiel 1b)

### I Herstellung der Katalysatorphase und Präformierung

60 mg (0,233 mmol) Rhodium(III)acetat werden in 39 ml einer 0,6 M wäßrigen Lösung von Trinatrium-tri(m-sulfophenyl)phosphin (Na-TPPTS) gelöst und zu 21 ml entgastem Polyethylenglykol 400 (PEG 400) unter Rühren gegeben (Erwärmung!). Diese Katalysatorphase wird unter Stickstoffstrom in einen 200 ml Stahlautoklav gegeben und unter Rühren bei 25 bar Synthesegasdruck (CO/H₂ = 1/1) während 3 Stunden auf 125 °C erwärmt.

### II Hydroformylierung

Zu der präformierten Katalysatorlösung aus I werden analog Beispiel 1a) 30 ml (240 mmol) 1-Hexen zugegeben. Die Hydroformylierung wird analog Beispiel 1a) bei 125 °C und bei 30 bar Synthesegas durchgeführt. Die Zusammensetzung der Produktphase wird mittels Gaschromatographie und ¹H-NMR-Spektroskopie ermittelt und aus der Zusammensetzung der Umsatzgrad und die Selektivität ermittelt. Der Edelmetallaustrag in die organische Phase wird bestimmt. Die Ausbeute an Hydroformylierungsprodukt beträgt 82,6 %, das n/iso-Verhältnis beträgt 94 : 6. Die organische Phase enthält < 0,5 ppm Rh. (Beispie 1b) in Tabelle 1)

### Beispiel 1c) (Vergleichsversuch zu Beispiel 1d) ohne Zusatz von Polyethylenglykol)

Es wird verfahren wie in Beispiel 1a) jedoch mit dem Unterschied. daß bei der Hydroformylierungsreaktion ein Reaktionsdruck von 50 bar angewendet wird. Nach 3 Stunden Reaktionszeit wird die Produktphase wie oben beschrieben abgetrennt und analysiert. Die Ausbeute an Hydroformylierungsprodukten beträgt 39,2 %, das n/iso-Verhältnis beträgt 97 : 3. Die organische Phase enthält < 0,03 ppm Rh. (Beispiel 1c) in Tabelle 1)

### Beispiel 1d)

Es wird verfahren wie in Beispiel 1b), d. h. die Katalysatorphase setzt sich aus 60 mg Rhodium(III)acetat, 39 ml einer 0,6 M Lösung von Trinatrium-tri(msulfophenyl)phosphin (Na-TPPTS) und 21 ml entgastem Polyethylenglyko! 400 (PEG 400) zusammen. Die Präformierungsbedingungen sind identisch. Bei der Hydroformylierungsreaktion wird jedoch ein Reaktionsdruck von 50 bar angewendet. Nach 3 Stunden Reaktionszeit wird die Produktphase wie oben beschrieben abgetrennt und analysiert. Die Ausbeute an Hydroformylierungsprodukten beträgt 94,7 %, das n/iso-Verhältnis beträgt 89 : 11. Die organische Phase enthält 0.08 ppm Rh, (Beispiel 1d) in Tabelle 1)

### Beispiel 1e) (Vergleichsversuch zu Beispiel 1f) ohne Zusatz von Polyethyienglykol)

Es wird verfahren wie in Beispiel 1a) jedoch mit dem Unterschied. daß bei der Hydroformylierungsreaktion ein Reaktionsdruck von 80 bar angewendet wird. Nach 3 Stunden Reaktionszeit wird die Produktphase wie oben beschrieben, abgetrennt und analysiert. Die Ausbeute an Hydroformylierungsprodukten beträgt 36,2 %, das n/iso-Verhältnis beträgt 96 : 4 Die organische Phase enthält < 0,5 ppm Rh.
(Beispiel 1e) in Tabelle 1)

### Beispiel 1f)

Es wird verfahren wie in Beispiel 1b), d. h. die Katalysatorphase wird aus 60 mg Rhodium(III)acetat, 39 ml einer 0,6 M wäßrigen Lösung von Trinatrium-(msulfophenyl)phosphin (Na-TPPTS) und 21 ml entgastem Polyethylenglykol 400 (PEG 400) angesetzt und unter den oben beschriebenen Bedingungen präformiert.

Bei der Hydroformylierungsreaktion wird jedoch ein Reaktionsdruck von 80 bar angewendet. Nach 3 Stunden Reaktionszeit wird die Produktphase wie oben beschrieben abgetrennt und analysiert. Die Ausbeute an Hydroformylierungsprodukten beträgt 93,5 %, das n/iso-Verhältnis beträgt 79 : 21. Die organische Phase enthält 0,39 ppm Rh. (Beispiel 1f) in Tabelle 1)

### Beispiele 1g) bis 1p)

Die weiteren Beispiele 1g) - 1p) ) zur Zweiphasen-Hydroformylierung von 1-Hexen werden analog durchgeführt. Zu einer Lösung von 60 mg Rhodium(III)acetat in 39 ml einer 0,6 M wäßrigen Lösung von Trinatrium-tri(m-sulfophenyl)phosphin (Na-TPPTS) wird die in der Tabelle 1 spezifizierte Menge eines Polyethylenglykols 200, 400 bzw. 600 (PEG 200, PEG 400 bzw. PEG 600) bzw. Alkylethoxylats (Genapol T 250: Tetradecylpolyethylenglykolether mit 26 Ethoxyeinheiten) zugesetzt, die Lösung auf 60 ml Gesamtvolumen mit entgastem, destillierten Wasser aufgefüllt und analog Beispiel 1a) präformiert. Nach Zusatz von 30 ml 1-Hexen gemäß Beispiel 1a) wird die Hydroformylierungsreaktion bei dem in Tabelle 1 angegebenen Reaktionsdruck durchgeführt. Die Reaktionsbedingungen, die Ausbeute an Hydroformylierungsprodukten und die anderen analytischen Daten sowie der Rhodiumgehalt der organischen Phase sind in der Tabelle 1 zusammengefaßt.

### Beispiel 1p) (Vergleichsversuch zu den Beispielen 1g), 1n) und 1o) ohne Zusatz von Polyethylenglykol)

In Beispiel 1p) wird anstelle von Polyethylenglykol 200, 400 oder 600 als Additiv Genapol T 250® C₁₂H₂₅(OCH₂CH₂)₂₆OH verwendet, ein Alkoholethoxylat mit einer 12 C-Atome enthaltenden Alkylkette und 26 Ethoxylateinheiten und die Katalysatorlösung in gewohnter Weise präformiert. Die Hydroformylierung wird nach Zusatz von 30 ml (240 mmol) 1-Hexen gemäß Beispiel 1c) durchgeführt. Nach Beendigung der Reaktion erhält man eine dunkelbraune Lösung. die sich nicht, wie gemäß DE 31 35 127 zu erwarten wäre, in eine organische und eine polare Katalysatorphase trennt. Nach dem Abkühlen verbleibt eine hellgelbe dickflüssige Reaktionsmasse, in der sich auch nach 24 Stunden keine Produkphase ausbildet. Eine gaschromatographische Analyse des Reaktionsprodukts ist nicht möglich. Aus dem Synthesegasverbrauch errechnet sich ein Umsatz von ungefähr 45 %. (Wie Beispiel 1 p) beweist, eignen sich Alkoholethoxylate mit längeren Alkylketten. die als nichtionische Tenside kommerziell Verwendung finden, nicht für die durchgeführte Zweiphasen-Hydroformylierung.) Die Reaktionsbedingungen und die analytischen Daten sind für die Beispiele 1 a) bis 1 p) in der nachfolgenden Tabelle zusammengestellt.

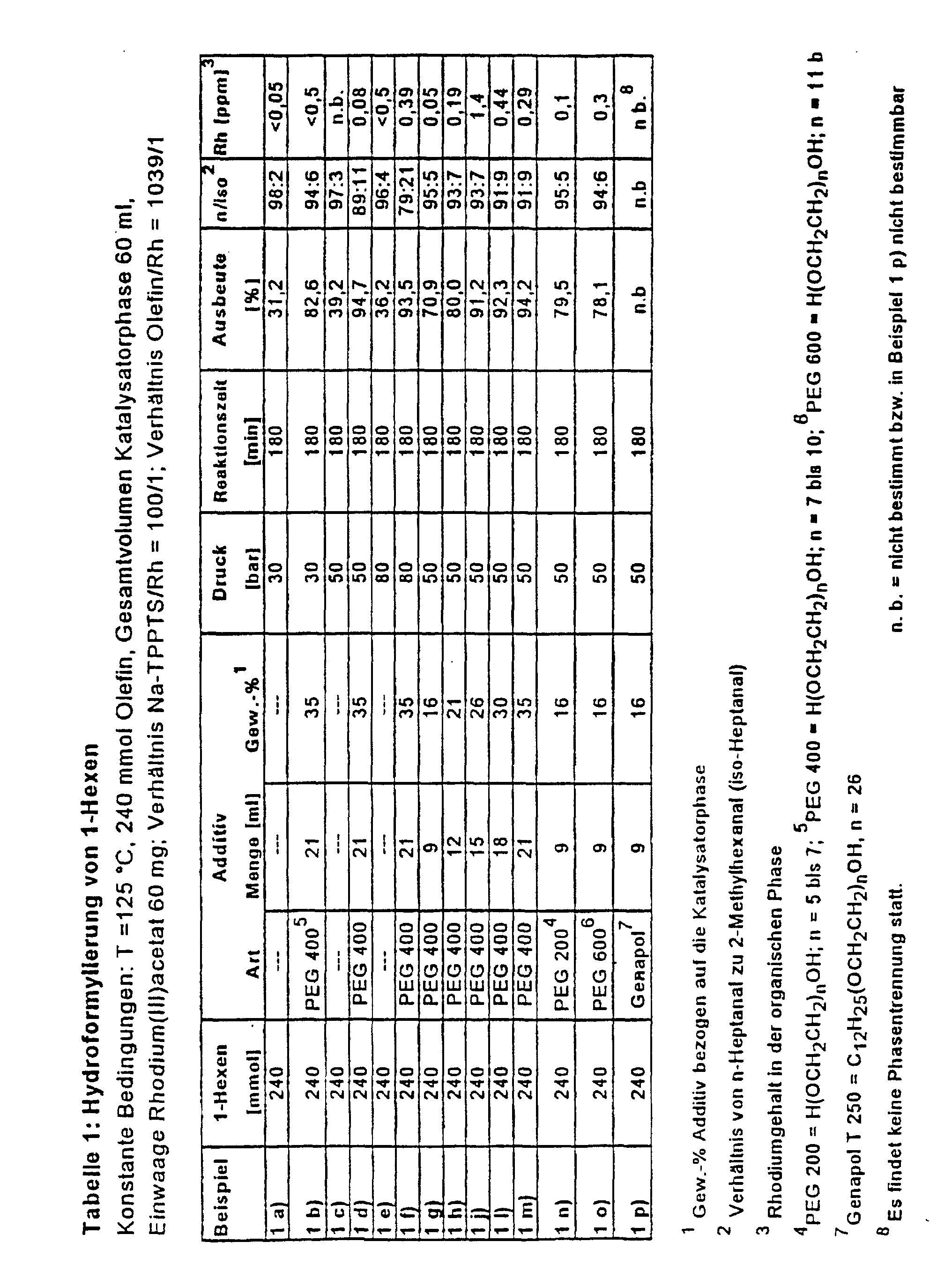

### 2. Hydroformylierung von 1-Octen

### Beispiel 2a) (Vergleichsversuch zu Beispiel 2b) ohne Zusatz von Polyethylenglykol)

### I Herstellung der Katalysatorphase und Präformierung

60 mg (0,233 mmol) Rhodium(III)acetat werden in 39 ml einer 0,6 M wäßrigen Lösung von Trinatrium-tri(m-sulfophenyl)phosphin (Na-TPPTS) entsprechend einem Molverhältnis Rhodium zu Ligand von 1 : 100 und 21 ml entgastem destillierten Wasser gelöst und in einen 200 ml Stahlautoklav unter Stickstoffstrom gegeben. Die so hergestellte Katalysatorlösung wird unter Rühren bei 25 bar Synthesegasdruck (CO/H₂ = 1/1) während 3 Stunden auf 125 °C erwärmt, wobei der aktive Katalysatorkomplex entsteht und die Lösung eine gelbe Farbe annimmt.

### II Hydroformylierung

Zu der präformierten Katalysatorlösung aus I werden bei einem Reaktionsdruck von 30 bar und bei 125 °C 37,7 ml (240 mmol) 1-Octen über einen vorgeschalteten 200 ml Stahlautoklav mit leichtem Überdruck zugegeben. Das Verhältnis von Olefin zu Rhodium beträgt wie vorher 1039 : 1. Die Hydroformylierungsreaktion wird durch Einschalten des Magnetrührers in Gang gesetzt. Während einer Reaktionszeit von 3 Stunden wird die Temperatur auf 125 °C gehalten und der Reaktionsdruck in einem Druckband von +/- 2 bar durch manuelles Zudosieren von Synthesegas konstant gehalten. Nach Ablauf von 3 Stunden werden Rührer und Heizung abgeschaltet, der Autoklav wird auf 40 bis 50 °C abgekühlt und nach einer Absetzzeit von 30 bis 60 Minuten die obere Produktphase von der Katalysatorphase mittels Phasentrennung in einem Scheidetrichter getrennt. Produktphase und Katalysatorphase werden gewogen. Die Zusammensetzung der Produktphase wird mittels Gaschromatographie und ¹H-NMR-Spektroskopie ermittelt und aus der Zusammensetzung der Umsatzgrad und die Selektivität ermittelt. Der Rhodiumgehalt der organischen Phase wird elementaranalytisch mittels Graphitrohr-Atomabsorptionsspektrometrie nach Probenaufschluß bestimmt. Die Ausbeute an Hydroformylierungsprodukt beträgt 9,1 %, das n/iso-Verhältnis beträgt 98 : 2. Die organische Phase enthält < 0,05 ppm Rh. (Beispiel 2a) in Tabelle 2)

### Beispiel 2b)

### I Herstellung der Katalysatorphase und Präformierung

60 mg (0,233 mmol) Rhodium(III)acetat werden in 39 ml einer 0.6 M wäßrigen Lösung von Trinatrium-tri(m-sulfophenyl)phosphin (Na-TPPTS) gelöst und zu 21 ml entgastem Polyethylenglykol 400 unter Rühren gegeben (Erwärmungl). Diese Katalysatorphase wird unter Stickstoffstrom in einen 200 ml Stahlautoklav gegeben und bei 25 bar Synthesegasdruck (CO/H₂ = 1/1) während 3 Stunden auf 125 °C erwärmt.

### II Hydroformylierung

Zu der präformierten Katalysatorlösung aus I werden, wie in Beispiel 2b) beschrieben, 37,7 ml (240 mmol) 1-Octen bei 30 bar und bei 125 °C zugegeben. Die Hydroformylierungsreaktion wird analog Beispiel 2 a) durchgeführt. Nach Ablauf von 3 Stunden werden Rührer und Heizung abgeschaltet, der Autoklav wird auf 40 bis 50 °C abgekühlt und nach einer Absetzzeit von 30 bis 60 Minuten die obere Produktphase von der Katalysatorphase mittels Phasentrennung in einem Scheidetrichter getrennt. Produktphase und Katalysatorphase werden gewogen und die Zusammensetzung der Produktphase wie oben beschrieben analysiert.
Die Ausbeute an Hydroformylierungsprodukten (n- und iso-Nonanal, Spuren von Nonanol und Pelargonsäure) beträgt 58,9 %, das Verhältnis von n-Nonanal zu 2-Methyloctanal beträgt 92 : 8. Die organische Phase enthält < 0,5 ppm Rh. (Beispiel 2b) in Tabelle 2)

### Beispiel 2c) (Vergleichsversuch zu Beispiel 2d) ohne Zusatz einer Verbindung der Formel (1))

Es wird verfahren wie in Beispiel 2a) jedoch mit dem Unterschied, daß bei der Hydroformylierungsreaktion I ein Reaktionsdruck von 50 bar angewendet wird. Nach 3 Stunden Reaktionszeit wird die Produktphase wie oben beschrieben abgetrennt und analysiert. Die Ausbeute an Hydroformylierungsprodukten beträgt 7,6 %, das n/iso-Verhältnis beträgt 95 : 5. Die organische Phase enthält < 0,03 ppm Rh. (Beispiel 2c) in Tabelle 2)

### Beispiel 2d)

Es wird verfahren wie in Beispiel 2b) jedoch mit dem Unterschied. daß bei der Hydroformylierungsreaktion ein Reaktionsdruck von 50 bar angewendet wird. Nach 3 Stunden Reaktionszeit wird die Produktphase wie oben beschrieben abgetrennt und analysiert. Die Ausbeute an Hydroformylierungsprodukten beträgt 78,9 %, das n/iso-Verhältnis beträgt 90 : 10. Die organische Phase enthält 0,2 ppm Rh. (Beispiel 2d) in Tabelle 2)

### Beispiel 2e) (Vergleichsversuch zu Beispiel 2f) ohne Zusatz von Polyethylenglykol)

Es wird verfahren wie in Beispiel 2a), d. h. die Zusammensetzung der Katalysatorphase und die Präformierungsbedingungen sind identisch. Bei der Hydroformylierungsreaktion wird jedoch ein Reaktionsdruck von 80 bar angewendet. Nach 3 Stunden Reaktionszeit wird die Produktphase wie oben beschrieben abgetrennt und analysiert. Die Ausbeute an Hydroformylierungsprodukten beträgt 7,9 %, das n/iso-Verhältnis beträgt 95:5. Die organische Phase enthält < 0,5 ppm Rh. (Beispiel 2e) in Tabelle 2)

### Beispiel 2f)

Es wird verfahren wie in Beispiel 2b), d. h. die Katalysatorphase setzt sich aus 60 mg Rhodium(III)acetat, 39 ml einer 0,6 M wäßrigen Lösung von Trinatrium-tri(msulfophenyl)phosphin und 21 ml entgastem Polyethylenglykol 400 zusammen. Die Präformierungsbedingungen sind identisch. Bei der Hydroformylierungsreaktion wird jedoch ein Reaktionsdruck von 80 bar angewendet. Nach 3 Stunden Reaktionszeit wird die Produktphase wie oben beschrieben abgetrennt und analysiert. Die Ausbeute an Hydroformylierungsprodukten beträgt 76,2 %, das n/iso-Verhältnis beträgt 85 : 15. Die organische Phase enthält 0.04 ppm Rh. (Beispiel 2f) in Tabelle 2)

### Beispiele 2g) bis s)

Die weiteren Beispiele (Beispiel 2g) bis 2s)) zur Zweiphasen-Hydroformylierung von 1-Octen (siehe auch Tabelle 2) werden in analoger Weise durchgeführt. Zu einer Lösung von 60 mg Rhodium(III)acetat in 39 ml einer 0,6 M wäßrigen Lösung von Trinatrium-tri(m-sulfophenyl)phosphin (Na-TPPTS) wird die in der Tabelle 2 spezifizierte Menge von Polyethylenglykol 400, Triethylengkykol. eines Alkoholethoxylats (M 350, 450 PR) bzw. Brij 35® (C₁₂H₂₅(OCH₂CH₂)₂₃OH zugesetzt. Die Katalysatorlösung wird, mit entgastem, destillierten Wasser, falls das Volumen der Lösung kleiner 60 ml ist, auf 60 ml Gesamtvolumen aufgefüllt. Die Katalysatorlösung wird analog Beispiel 2 a) präformiert. Nach Zusatz von 37,7 ml 1-Octen werden die Hydroformylierungsreaktionen bei den angegebenen Reaktionsdrucken durchgeführt. Die Ausbeuten an Hydroformylierungsprodukten sowie der Rhodiumgehalt der organischen Phase sind in der Tabelle 2 zusammengefaßt.

### Beispiel 2I) Vergleichsversuch analog Beispiel 78 der DE 31 35 127 jedoch unter Verwendung von Na-TPPTS anstelle von Natrium Triphenylphosphinmonosulfonat (3-Ph₂PC₆H₅SO₃Na) (Na-TPPMS) und Ver-wendung von 1-Octen anstelle von 1-Dodecen

Beispiel 2l) wird analog Beispiel 78 aus der DE 31 35 127, d. h. mit den gleichen Mengenverhältnissen der Einsatzstoffe und mit 1-Octen als Olefin durchgeführt. Nach Präformierung der Katalysatorphase bestehend aus 46 mg Rhodium(III)acetat, 1,04 g Brij 35® (C₁₂H₂₅(OCH₂CH₂)₂₃OH) entsprechend einem Verhältnis von Additiv zu Rhodium von 5 zu 1, 0,87 ml einer 0,6 M Lösung von Trinatrium-tri(msulfophenyl)phosphin (Na-TPPTS) entsprechend einem Verhältnis von TPPTS zu Rhodium von 3 zu 1, und 60 ml Pufferlösung pH = 10 (KHCO₃ - KOH) ml 21,2 ml (135 mmol) 1-Octen in gewohnter Weise zudosiert und bei einem Reaktionsdruck von 50 bar hydroformyliert. Nach Abkühlen der Reaktionsmasse auf 25 °C verbleibt eine braune, stark schäumende, trübe Reaktionslösung, die sich auch nach längerem Stehen nicht mehr in zwei Phasen trennt.
Durch Extraktion der gesamten, gelblich gefärbten Reaktionsmasse mit 20 ml Pentan gelingt es, einen Teil der organischen Phase zu isolieren. Durch gaschromatographische Analyse wird ein Umsatzgrad von 22,8 % bestimmt. (Beispiel 2l) in Tabelle 2)

### Beispiel 2m) (Vergleichsversuch unter Verwendung von Brij 35® anstelle von PEG 400)

Beispiel 2l) wird wiederholt, wobei jetzt 46 mg Rhodium(III)acetat. 30,0 g Brij 35® , 29 ml einer 0,6 M Lösung von Trinatrium-tri(m-sulfophenyl)phosphin (Na-TPPTS) entsprechend einem Verhältnis von TPPTS zu Rhodium von 100 zu 1 angesetzt werden und präformiert werden. Anschließend werden 21,2 ml (135 mmol) 1-Octen in gewohnter Weise zudosiert und die Hydroformylierung bei 50 bar Druck durchgeführt. Bei diesem Hydroformylierungsansatz wird nach Abkühlen eine zitronengelbe Paste erhalten, die keine Phasentrennung zeigt. Eine Extraktion mit Pentan gelingt nicht. (Beispiel 2m) in Tabelle 2)
Die Beispiele 2I) und 2m) zeigen, daß sich Brij 35® in Kombination mit TPPTS nicht für die Zweiphasen-Hydroformylierung eignet, da nach der Hydroformylierungsreaktion keine Phasentrennung der ursprünglichen wäßrigen und der organischen Phase stattfindet.

### Beispiel 2k)

Beispiel 2m) wird wiederholt, jedoch mit dem Unterschied, daß anstelle von 30 g Brij 35® die gleiche Menge PEG 400 eingesetzt wird. Im Unterschied zu 2m) findet bei diesem Hydroformylierungsansatz eine Phasentrennung statt. Nach Abtrennen der organischen Phase wird die Ausbeute an Hydroformylierungsprodukten gaschromatographisch bestimmt. Sie beträgt 90,2 %. Das nliso-Verhältnis ist 82 :
18. (Beispiel 2k) in Tabelle 2)

### Beispiel 2p) Vergleichsversuch unter Verwendung eines gemischten Polyalkylenglykols der Formel CH₃[(OCH₂CH₂)₄(OCH₂CHCH₃)]₅OH (Handelsbezeichnung M 41/40 von Hoechst, MG ca. 1000)

### Beispiel 2b) wird wiederholt, jedoch mit dem Unterschied, daß anstelle von 21 ml PEG 400 die gleiche Menge des Polyalkylenglykols

CH₃[(OCH₂CH₂)₄(OCH₂CHCH₃)]₅OH (Handelsprodukt der Hoechst AG;
Bezeichnung M 41/40, Molgewicht ungefähr 1000 g/mol) eingesetzt wird. Nach Abtrennen der organischen Phase wird die Ausbeute an Hydroformylierungsprodukten gaschromatographisch bestimmt. Sie beträgt 23,0 %. Das n/iso-Verhältnis ist 82 : 8 (Beispiel 2p) in Tabelle 2). Beispiel 2p) beweist, daß gemischte Glykole mit einem Anteil von Propylenglykoleinheiten von 20 % im Vergleich zu den reinen Polyethylenglykolen schlechtere Ergebnisse zeigen.

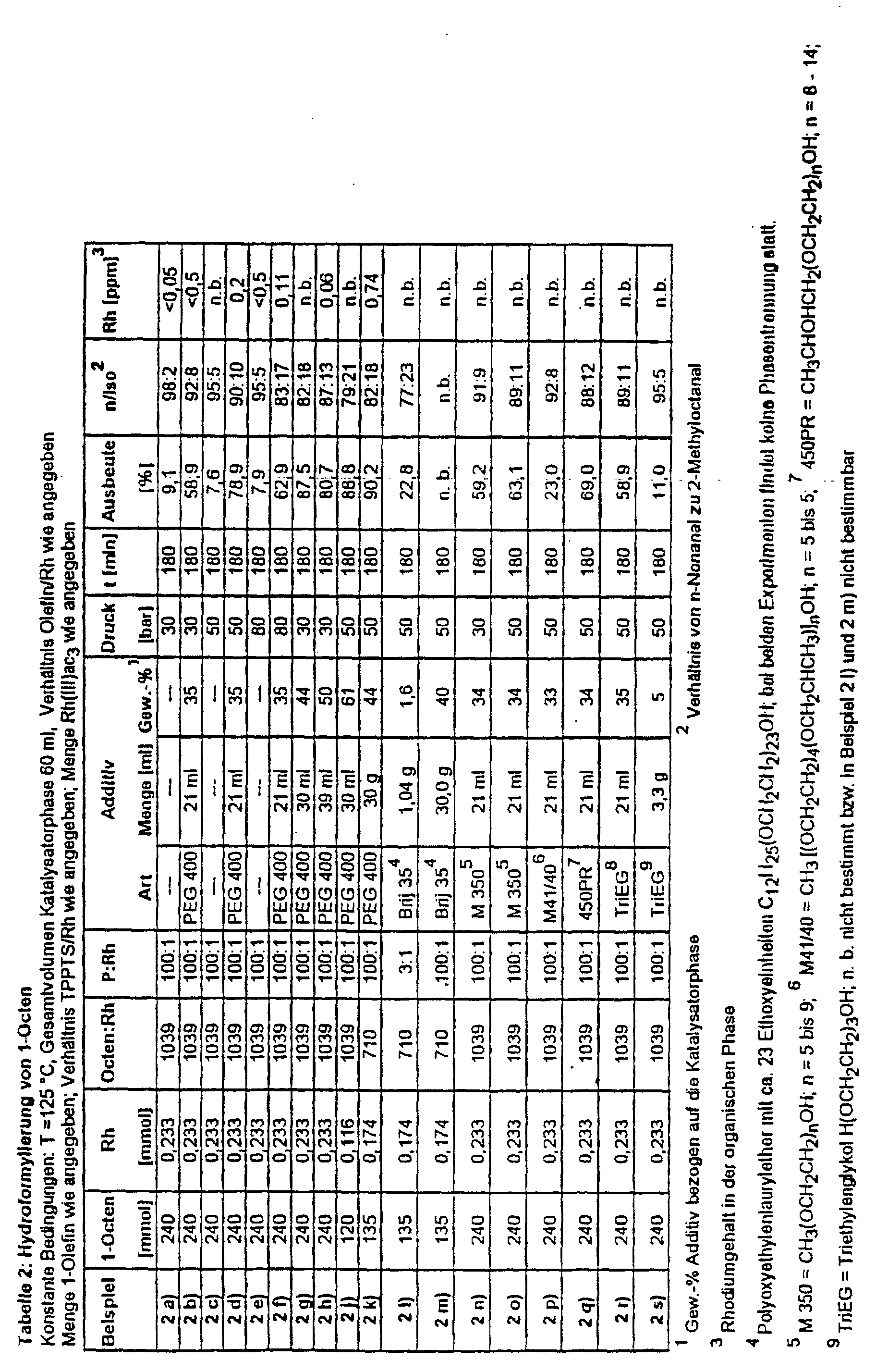

### 3. Reihenbeispiel zur Zweiphasen-Hydroformylierung von 1-Octen

### Beispiele 3a) bis 3i) (Wiedereinsatz der wäßrigen Katalysatorphase)

Es wurde eine Beispielsreihe zur Zweiphasen-Hydroformylierung von 1-Octen durchgeführt, bei der die Katalysatorphase insgesamt 8 Mal eingesetzt wurde, um zu demonstrieren, daß sich Katalysatorphase ohne Beeinträchtigung der katalytischen Aktivität wiederverwenden läßt. Das der erste Beispiel der Beispielsreihe wurde analog Beispiel 2f) durchgeführt. Nach 3 Stunden Hydroformylierungszeit wurde der Autoklav auf 50 °C abgekühlt und die obere organische Phase mittels eines Steigrohrs, welches in das Hydroformylierungsprodukt eintaucht und oberhalb der Phasengrenze endet, mit leichtem Überdruck aus dem Autokiaven in einen Vorlageautoklaven gedrückt. In den Beispielen 3b) bis 3i) wurde jeweils die gleiche Katalysatorphase des jeweils vorangegangenen Beispiels verwendet, indem jeweils 37,7 ml 1-Octen zu der Katalysatorphase in bekannter Weise zugegeben wurden, die Hydrofomylierungsreaktion durchgeführt und die organische Phase wie oben beschrieben, abgetrennt wurde.
Die Reaktionsbedingungen, die erzielten Ausbeuten, die n-/iso-Verhältnisse und der Rhodiumgehalt in der organischen Phase sind in Tabelle 3 zusammengefaßt.

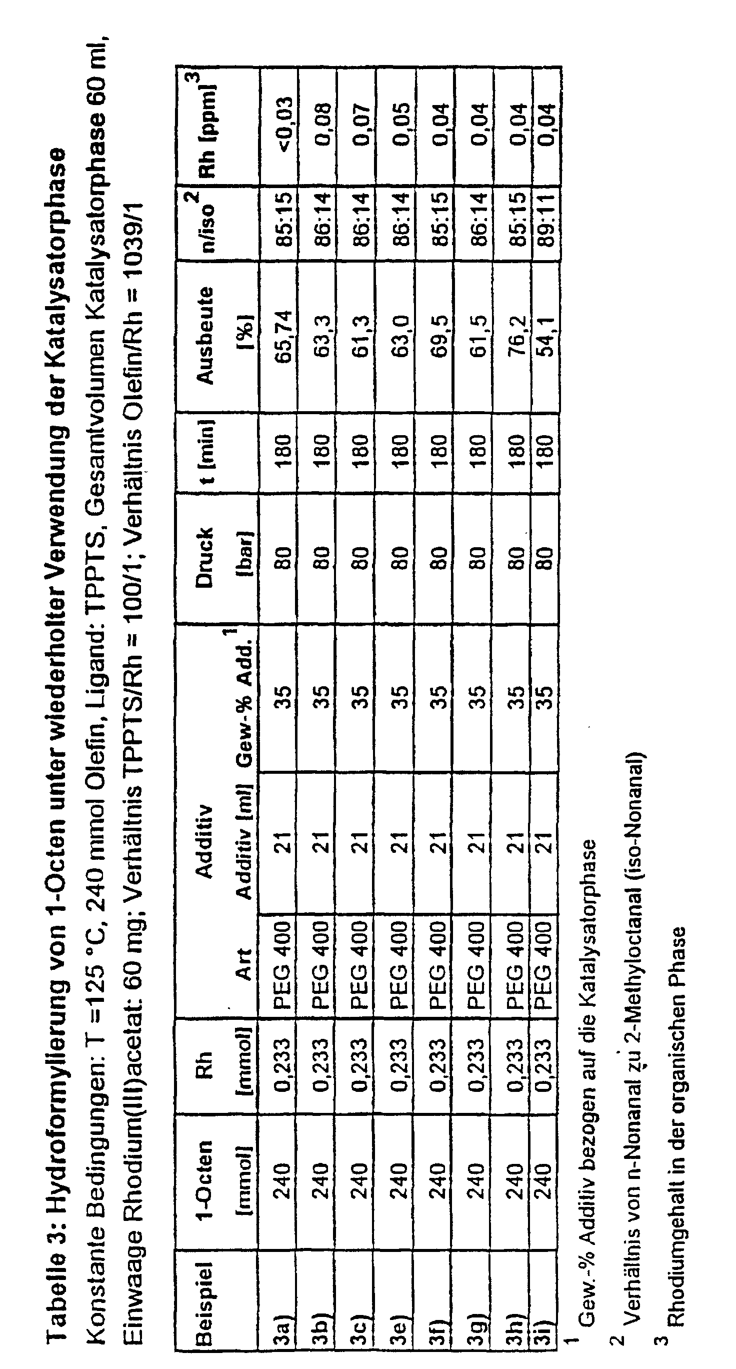

### 4. Hydroformylierung von 4-Vinylcyclohexen

### Beispiel 4a) (Vergleichsversuch zu Beispiel 4b) und 4c) ohne Zusatz von Polyethylenglykol)

### I Herstellung der Katalysatorphase und Präformierung

Die Katalysatorphase wird analog Beispiel 1a) hergestellt und präformiert.

### II Hydroformylierung

Zu der präformierten Katalysatorlösung werden 31,3 ml (240 mmol) 4-Vinylcyclohexen, wie zuvor beschrieben, zudosiert und die Hydroformylierungsreaktion bei einem Reaktionsdruck von 80 bar und bei 125 °C durchgeführt. Nach der Phasentrennung wird die Produktphase in bekannter Weise analysiert. Die Ausbeute an Hydroformylierungsprodukt beträgt 60,8 %, das n/iso-Verhältnis beträgt 97 : 3. Die organische Phase enthält 0,03 ppm Rh. (Beispiel 4a) in Tabelle 4)

### Beispiel 4b)

Die Katalysatorphase wird aus 60 mg Rhodium(III)acetat, 39 ml einer 0,6 M Lösung von Trinatrium-tri(m-sulfophenyl)phosphin (Na-TPPTS), 11 ml entgastem Polyethylenglykol 400 und 10 ml entgastem Wasser angesetzt und wie zuvor beschrieben präformiert. Zu der präformierten Katalysatorlösung werden 31,3 ml 4-Vinylcyclohexen (240 mmol) wie zuvor beschrieben zudosiert und die Hydroformylierungsreaktion bei einem Reaktionsdruck von 80 bar und bei 125 °C durchgeführt. Die Reaktion ist bereits nach 150 min beendet. Nach der Phasentrennung wird die Produktphase analysiert. Die Ausbeute an Hydroformylierungsprodukt beträgt 96,7 %, das n/iso-Verhältnis beträgt 94 : 6. Die organische Phase enthält 0,19 ppm Rh. (Beispiel 4b) in Tabelle 4)

### Beispiel 4c)

Die Katalysatorphase wird aus 60 mg Rhodium(III)acetat, 39 ml einer 0,6 M Lösung von Trinatrium-tri(m-sulfophenyl)phosphin (Na-TPPTS), 5 ml entgastem Polyethylenglykol 400 und 16 ml entgastem Wasser angesetzt und wie oben beschrieben präformiert. Zu der präformierten Katalysatorlösung werden 31,3 ml 4-Vinylcyclohexen (240 mmol), wie zuvor beschrieben, zudosiert und die Hydroformylierungsreaktion bei einem Reaktionsdruck von 80 bar und bei 125 °C durchgeführt. Die Reaktion ist bereits nach 2,5 Stunden beendet, die Phasentrennung wird durchgeführt und die Produktphase wie gewohnt analysiert. Die Ausbeute an Hydroformylierungsprodukt beträgt 92,3 %, das n/iso-Verhältnis beträgt 95 : 5. Die organische Phase enthält 0,15 ppm Rh. (Beispiel 4 c) in Tabelle 4)

### Beispiel 4d)

Beispiel 4c) wird wiederholt, mit dem Unterschied, daß bei der Hydroformylierungsreakion ein Druck von 50 bar angewendet wird und die Reaktionszeit auf 3 Stunden erhöht wird. Die Ausbeute an Hydroformylierungsprodukt beträgt 87,6 %, das n/iso-Verhältnis beträgt 97 : 3. Die organische Phase enthält 0,03 ppm Rh. (Beispiel 4d) in Tabelle 4)

### Beispiel 4e) (Vergleichsversuch zu den Beispielen 4d) und 4f) ohne Zusatz von Polyethylenglykol)

Die Katalysatorphase wird analog Beispiel 4a) angesetzt und präformiert. Zu der präformierten Katalysatorlösung werden 31,3 ml (240 mmol) 4-Vinylcyclohexen, wie zuvor beschrieben, zudosiert und die Hydroformylierungsreaktion bei einem Reaktionsdruck von 50 bar und bei 125 °C durchgeführt. Die Hydroformylierungsreaktion ist erst nach 500 min beendet, die Phasentrennung wird durchgeführt und die Produktphase wie gewohnt analysiert. Die Ausbeute an Hydroformylierungsprodukt beträgt 82,5 %, das n/iso-Verhältnis beträgt 98 : 2. Die organische Phase enthält 0,04 ppm Rh. (Beispiel 4e) in Tabelle 4)

### Beispiel 4f)

Die Katalysatorphase wird analog Beispiel 4c) angesetzt und präformiert. Zu der präformierten Katalysatorlösung werden 31,3 ml (240 mmol) 4-Vinylcyclohexen, wie zuvor beschrieben, zudosiert und die Hydroformylierungsreaktion bei einem Reaktionsdruck von 50 bar und bei 125 °C durchgeführt. Die Reaktion ist nach 4 Stunden beendet, die Phasentrennung wird durchgeführt und die Produktphase wie gewohnt analysiert. Die Ausbeute an Hydroformylierungsprodukt beträgt 95,8 %, das n/iso-Verhältnis beträgt 97 : 3. Die organische Phase enthält < 0,03 ppm Rh. (Beispiel 4f) in Tabelle 4). Die Reaktionsbedingungen und die analytischen Daten sind für die Beispiele 4a) bis 4f) in der nachfolgenden Tabelle 4 zusammengefaßt.

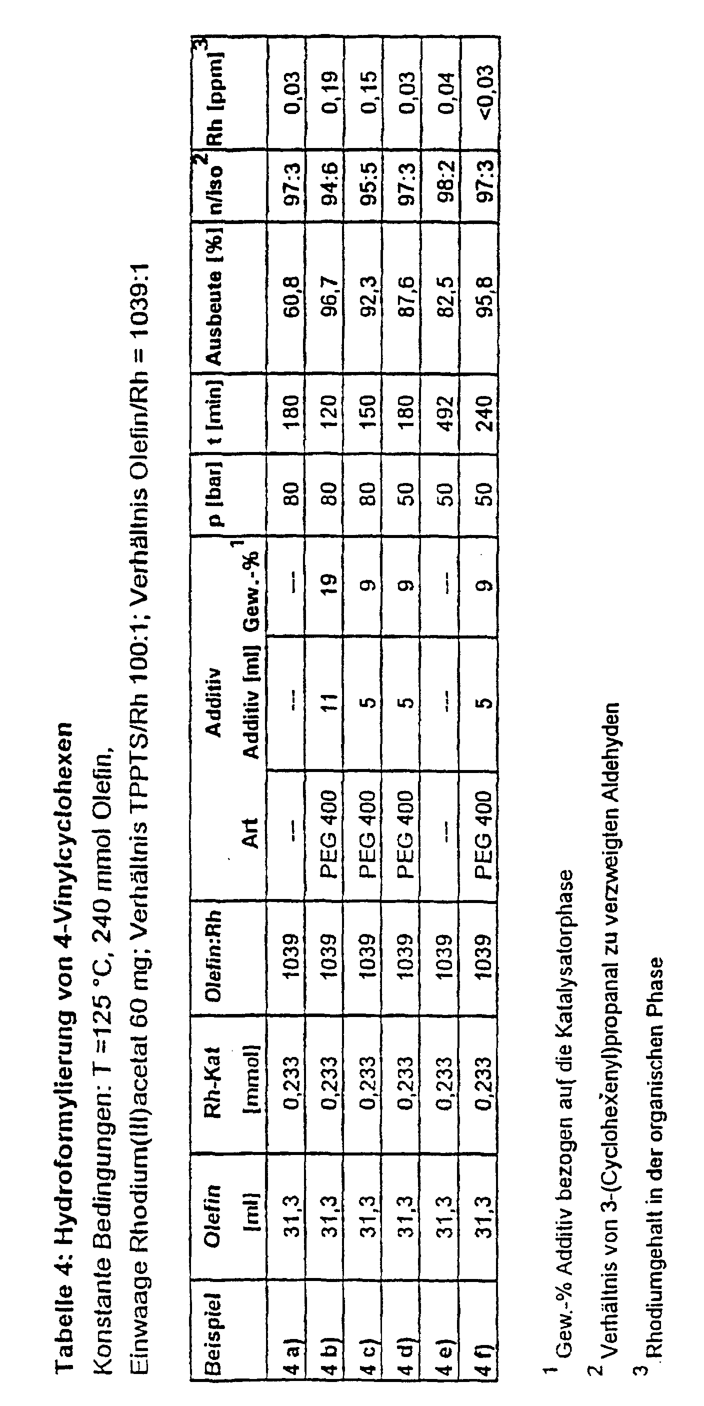

### 5. Hydroformylierung von 1-Decen

### Beispiel 5a) (Vergleichsversuch zu Beispiel 5b) ohne Zusatz von Polyethylenglykol)

### I Herstellung der Katalysatorphase und Präformierung

60 mg (0,233 mmol) Rhodium(III)acetat werden in 39 ml einer 0,6 M wäßrigen Lösung von Trinatrium-tri(m-sulfophenyl)phosphin (Na-TPPTS) entsprechend einem Molverhältnis Rhodium zu Ligand von 1 : 100 und 21 ml entgastem destillierten Wasser gelöst und in einen 200 ml Stahlautoklav unter Stickstoffstrom gegeben. Die so hergestellte Katalysatorlösung wird bei 25 bar Synthesegasdruck (CO/H₂ = 1/1) während 3 Stunden auf 125 °C erwärmt, wobei der aktive Katalysatorkomplex entsteht und die Lösung sich intensiv gelb färbt.

### II Hydroformylierung

Zu der präformierten Katalysatorlösung werden unter einem Reaktionsdruck von 30 bar und bei 125 °C 45,5 ml (240 mmol) 1-Decen über einen vorgeschalteten 200 ml Stahlautoklav mit leichtem Überdruck zugegeben. Das Verhältnis von Olefin zu Rhodium beträgt 1039 : 1. Die Hydroformylierungsreaktion wird analog den vorher beschriebenen Beispielen bei 30 bar Synthesegasdruck durchgeführt. Nach Ablauf von 3 Stunden werden Rührer und Heizung abgeschaltet, der Autoklav auf 40 bis 50 °C abgekühlt und die obere Produktphase von der Katalysatorphase mittels Phasentrennung in einem Scheidetrichter getrennt. Produktphase und Katalysatorphase werden gewogen. Die Zusammensetzung der Produktphase wird, wie vorher beschrieben, analysiert. Die Ausbeute an Hydroformylierungsprodukt beträgt 1,9 %, das n/iso-Verhältnis beträgt 100 : 0. Die organische Phase enthält < 0,03 ppm Rh. (Beispiel 5a) in Tabelle 5)

### Beispiel 5b)

Die Katalysatorphase wird analog Beispiel 1b) angesetzt und präformiert. Zu der präformierten Katalysatorlösung werden 95,5 ml (240 mmol) 1-Decen, wie vorher beschrieben, zudosiert und die Hydroformylierungsreaktion bei einem Reaktionsdruck von 30 bar und bei 125 °C durchgeführt. Nach der Phasentrennung wird die Produktphase analysiert:
Die Ausbeute an Hydroformylierungsprodukt beträgt 19,1 %, das n/iso-Vernältnis beträgt 93 : 7. Die organische Phase enthält < 0,03 ppm Rh. (Beispiel 5b) in Tabelle 5)

### Beispiel 5c) (Vergleichsversuch zu Beispiel 5d) ohne Zusatz von Polyethylenglykol)

Es wird verfahren wie in Beispiel 5a) mit dem Unterschied, daß bei der Hydroformylierungsreaktion ein Reaktionsdruck von 80 bar angewendet wird. Nach 3 Stunden Reaktionszeit wird die Produktphase, wie oben beschrieben, abgetrennt und analysiert. Die Ausbeute an Hydroformylierungsprodukten beträgt 2,7 %, das n/iso-Verhältnis beträgt 94 : 6. Die organische Phase enthält < 0.03 ppm Rh. (Beispiel 5c) in Tabelle 5)

### Beispiel 5d)

Es wird verfahren wie in Beispiel 5b) mit dem Unterschied, daß die Hydroformylierungsreaktion bei einem Druck von 80 bar durchgeführt wird. Nach 3 Stunden Reaktionszeit wird die Produktphase, wie oben beschrieben, abgetrennt und analysiert. Die Ausbeute an Hydroformylierungsprodukten beträgt 43,0 %, das n/iso-Verhältnis beträgt 87 : 13. Die organische Phase enthält 0,26 ppm Rh. (Beispiel 5d) in Tabelle 5).
Die Reaktionsbedingungen und die analytischen Daten sind für die Beispiele 5a) bis 5d) in der nachfolgenden Tabelle 5 zusammengestellt.

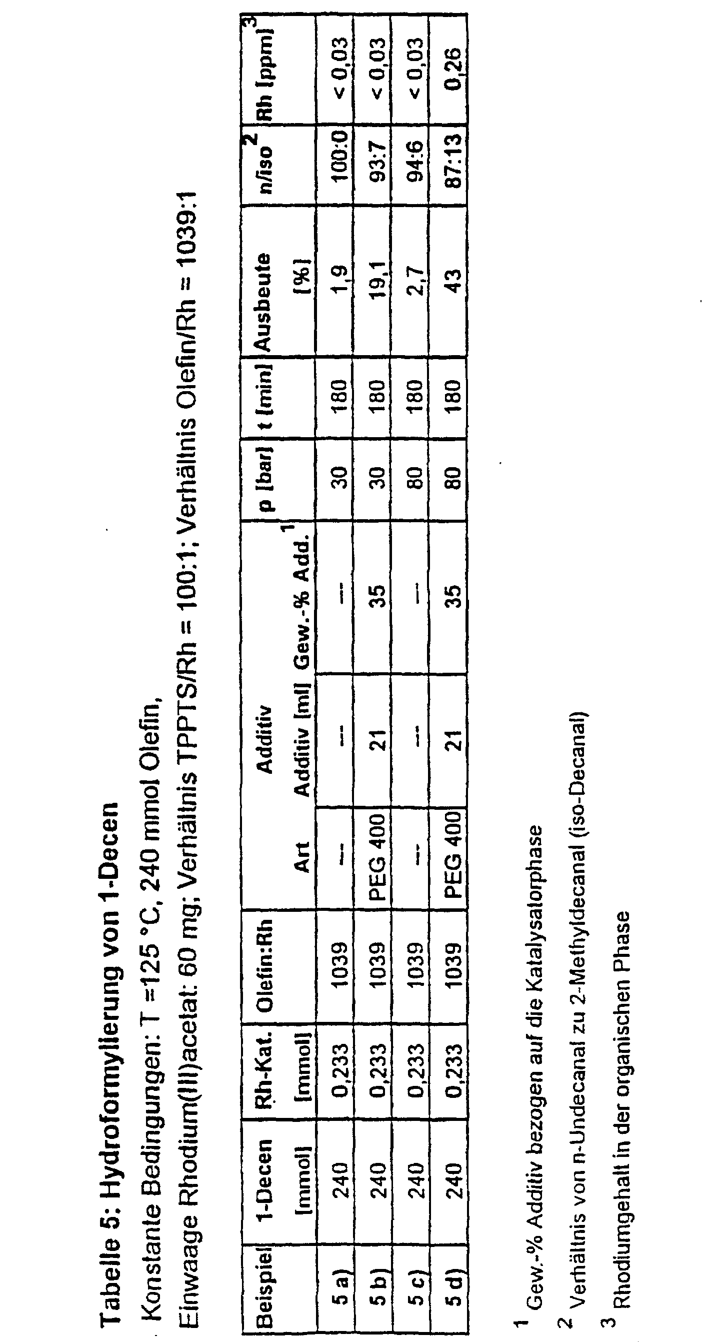

### 6. Hydroformylierung von 1-Dodecen

### Beispiel 6a) (Vergleichsversuch zu Beispiel 6b) ohne Zusatz von Polyethylenglykol)

Die Katalysatorphase wird analog Beispiel 1a) hergestellt und präformiert. Zu der präformierten Katalysatorlösung werden 53,3 ml (240 mmol) 1-Dodecen, wie zuvor beschrieben, zudosiert und die Hydroformylierungsreaktion 3 Stunden bei einem Reaktionsdruck von 30 bar und bei 125 °C durchgeführt. Nach der Phasentrennung wird die Produktphase analysiert. Die Ausbeute an Hydroformylierungsprodukt beträgt 0 %. Die organische Phase enthält < 0,03 ppm Rh. (Beispiel 6a) in Tabelle 6)

### Beispiel 6b)

Die Katalysatorphase wird analog 1b) angesetzt und wie oben beschrieben präformiert. Zu der präformierten Katalysatorlösung werden 53,3 ml (240 mmol) 1-Dodecen, wie vorher beschrieben, zudosiert und die Hydroformylierungsreaktion 3 Stunden bei einem Reaktionsdruck von 30 bar und bei 125 °C durchgeführt. Nach der Phasentrennung wird die Produktphase analysiert. Die Ausbeute an Hydroformylierungsprodukt beträgt 9,2 %, das n/iso-Verhältnis beträgt 87 : 13. Die organische Phase enthält < 0, 5 ppm Rh. (Beispiel 6b) in Tabelle 6)

### Beispiel 6c) (Vergleichsversuch zu Beispiel 6d) ohne Zusatz von Polyethylenglykol)

Die Katalysatorphase wird analog Beispiel 1a) hergestellt und präformiert. Zu der präformierten Katalysatorlösung werden 53,3 ml (240 mmol) 1-Dodecen wie vorher beschrieben zudosiert und die Hydroformylierungsreaktion 3 Stunden bei einem Reaktionsdruck von 80 bar und bei 125 °C durchgeführt. Nach der Phasentrennung wird die Produktphase analysiert. Die Ausbeute an Hydroformylierungsprodukt beträgt < 1 %. Das n/iso-Verhältnis ist aufgrund der niedrigen Ausbeute nicht bestimmbar. Die organische Phase enthält < 0,5 ppm Rh. (Beispiel 6c), Tabelle 6)

### Beispiel 6d)

Die Katalysatorphase wird analog 1 b) angesetzt und wie oben beschrieben präformiert. Zu der präformierten Katalysatorlösung werden 53.3 ml (240 mmol) 1-Dodecen, wie vorher beschrieben, zudosiert und die Hydroformylierungsreaktion 3 Stunden bei einem Reaktionsdruck von 80 bar und bei 125 °C durchgeführt. Nach der Phasentrennung wird die Produktphase analysiert.
Die Ausbeute an Hydroformylierungsprodukt beträgt 13,0 %, das n/iso-Verhältnis beträgt 75 : 25. Die organische Phase enthält 0,03 ppm Rh. (Beispiel 6d) in Tabelle 6)

### Beispiel 6e)

Die Katalysatorphase wird aus 60 mg Rhodium(III)acetat, 21 ml einer 0,6 M wäßrigen Lösung von Trinatrium-tri(m-sulfophenyl)phosphin (Na-TPPTS) und 39 ml PEG 400 angesetzt und präformiert. Das Verhältnis von TPPTS zu Rhodium beträgt hier 54 : 1. Die Hydroformylierungsreaktion wird 3 Stunden bei einem Reaktionsdruck von 80 bar und bei 125 °C durchgeführt. Nach der Phasentrennung wird die Produktphase analysiert. Die Ausbeute an Hydroformylierungsprodukt beträgt 76,4 %, das n/iso-Verhältnis beträgt 72 : 28. Die organische Phase enthält 0,52 ppm Rh. (Beispiel 6e) in Tabelle 6)

### Beispiel 6f)

Die Katalysatorphase wird aus 60 mg Rhodium(III)acetat, 15,5 ml einer 0,6 M wäßrigen Lösung von Trinatrium-tri(m-sulfophenyl)phosphin (Na-TPPTS) (9,32 mmol) und 40 ml PEG 400 angesetzt und präformiert. Das Verhältnis von TPPTS zu Rhodium beträgt hier 40 : 1. Die Hydroformylierungsreaktion wird 3 Stunden bei einem Reaktionsdruck von 50 bar und bei 125 °C durchgeführt. Nach der Phasentrennung wird die Produktphase analysiert. Die Ausbeute an Hydroformylierungsprodukt beträgt 59,0 %, das n/iso-Verhältnis beträgt 76 : 24. Die organische Phase enthält 0,08 ppm Rh. (Beispiel 6f) in Tabelle 6)
Die Reaktionsbedingungen und die analytischen Daten sind für die Beispiele 6a) bis 6f) in der nachfolgenden Tabelle 6 zusammengestellt.

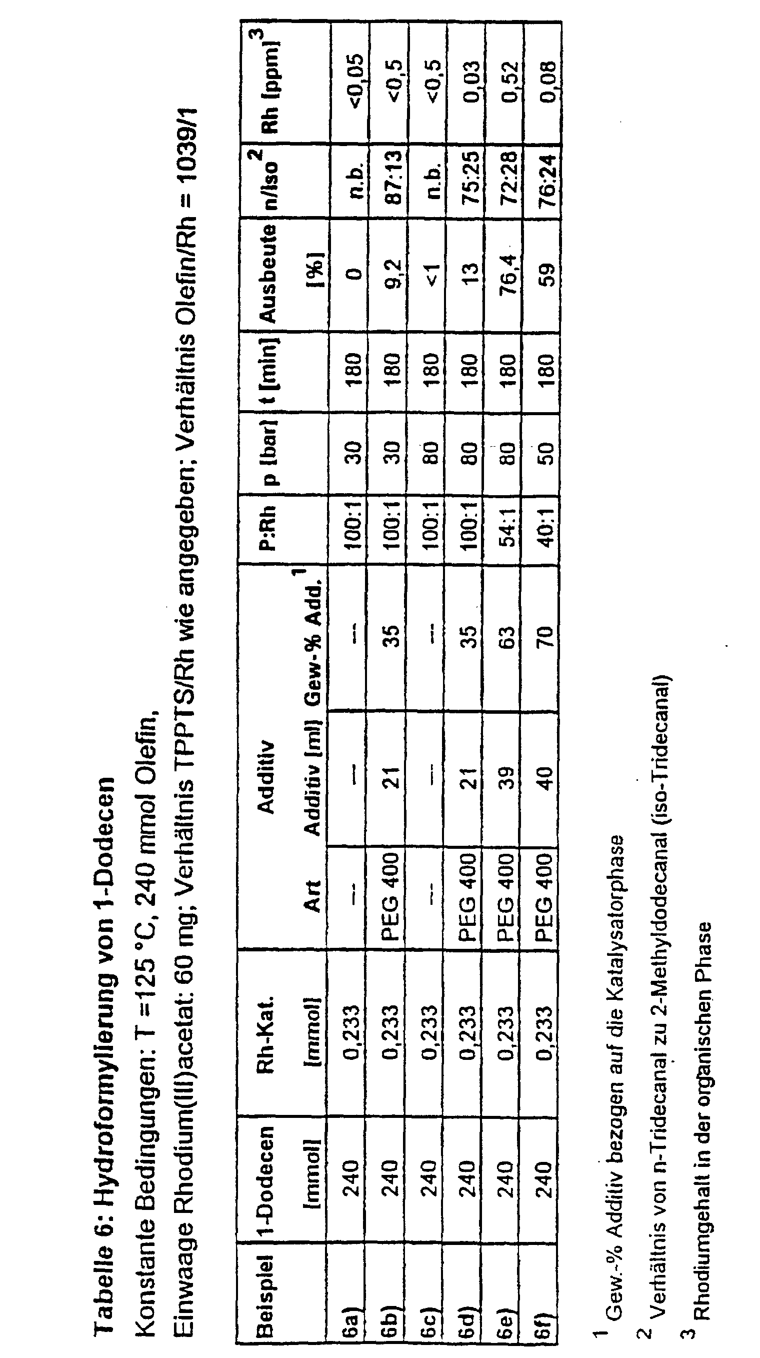

## Patentansprüche

1. Verfahren zur Herstellung von Aldehyden, bei dem man eine olefinisch ungesättigte Verbindung mit 6 bis 16 Kohlenstoffatomen mit Wasserstoff und Kohlenmonoxid in Anwesenheit von Lösungsvermittler und einer Rhodium und sulfonierte Triarylphosphine als Katalysator enthaltenden wäßrigen Phase umsetzt, **dadurch gekennzeichnet, daß** man in Anwesenheit einer wäßrigen Phase arbeitet, die mehr als 10 Gew.-% und bis zu 70 Gew.-% eines Gemisches von Verbindungen der Formel (1) R(OCH₂CH₂)ₙOR¹ enthält, wobei in der Formel (1) R für Wasserstoff, einen geradkettigen oder verzweigten Alkylrest mit 1 bis 4 Kohlenstoffatomen oder einen Hydroxyalkylrest mit 1 bis 4 Kohlenstoffatomen, R¹ für Wasserstoff oder einen Methylrest und n für eine ganze Zahl von 3 bis 50 steht, daß die Triarylphosphine wenigstens drei -(SO₃)M-Reste enthalten, worin M gleich oder verschieden ist und M für H, ein Alkalimetallion, ein Ammoniumion, ein quaternäres Ammoniumion, ein ½ Erdalkalimetallion oder ½ Zinkion steht und daß die Umsetzung bei 20 bis 170°C und 1 bis 300 bar stattfindet.

2. Verfahren nach Anspruch 1, **dadurch gekennzeichnet, daß** man als olefinisch ungesättigte Verbindung ein aliphatisches Olefin, cycloaliphatisches Olefin oder araliphatisches Olefin mit 6 bis 16 Kohlenstoffatomen einsetzt.

3. Verfahren nach Anspruch 1 oder 2, **dadurch gekennzeichnet, daß** man als olefinische Verbindung ein aliphatisches α-Olefin mit 6 bis 12 Kohlenstoffatomen einsetzt.

4. Verfahren nach einem oder mehreren der Ansprüche 1 bis 3, **dadurch gekennzeichnet, daß** man als trisulfonierte Triarylphosphine Verbindungen der Formel (2) 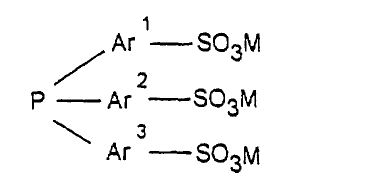 worin Ar¹, Ar² und Ar³ gleich oder verschieden sind und jeweils einen Phenyloder Naphthylrest bedeuten und M gleich oder verschieden ist und für ein Alkalimetallion, ein Ammoniumion, ein quaternäres Ammoniumion oder ein ½ Erdalkalimetallion oder ½ Zinkion steht, einsetzt.

5. Verfahren nach einem oder mehreren der Ansprüche 1 bis 4, **dadurch gekennzeichnet, daß** man als sulfonierte Triarylphosphine ein sulfoniertes Triphenylphosphin einsetzt.

6. Verfahren nach einem oder mehreren der Ansprüche 1 bis 4, **dadurch gekennzeichnet, daß** man als sulfoniertes Triarylphosphin Trinatrium-tri(msulfophenyl)phosphin einsetzt.

7. Verfahren nach einem oder mehreren der Ansprüche 1 bis 6, **dadurch gekennzeichnet, daß** man die wäßrige Phase entsprechend einer Menge von 2 x 10⁻⁶ bis 5 x 10⁻² Mol Rhodium pro Mol olefinische Verbindung einsetzt.

8. Verfahren nach einem oder mehreren der Ansprüche 4 bis 7, **dadurch gekennzeichnet, daß** man Rhodium und sulfonierte Triarylphosphine der Formel (2) im Molverhältnis 1 : 10 bis 1 : 1000 einsetzt.

9. Verfahren nach einem oder mehreren der Ansprüche 4 bis 8, **dadurch gekennzeichnet, daß** man Rhodium und sulfonierte Triarylphosphine der Formel (2) im Molverhältnis 1 : 50 bis 1 : 200 einsetzt.

10. Verfahren nach einem oder mehreren der Ansprüche 4 bis 9, **dadurch gekennzeichnet, daß** die wäßrige Phase bei Verwendung sulfonierter Triarlyphosphine der Formel (2) 100 bis 1000 ppm Rhodium enthält.

11. Verfahren nach einem oder mehreren der Ansprüche 4 bis 10, **dadurch gekennzeichnet, daß** die wäßrige Phase bei Verwendung sulfonierter Triarlyphosphine der Formel (2) 200 bis 500 ppm Rhodium enthält.

12. Verfahren nach einem oder mehreren der Ansprüche 4 bis 11, **dadurch gekennzeichnet, daß** die wäßrige Phase bei Verwendung sulfonierter Triarlyphosphine der Formel (2) 300 bis 400 ppm Rhodium enthält.

13. Verfahren nach einem oder mehreren der Ansprüche 1 bis 3, **dadurch gekennzeichnet, daß** man als sulfonierte Triarylphosphine Gemische der Verbindungen der Formel (3) 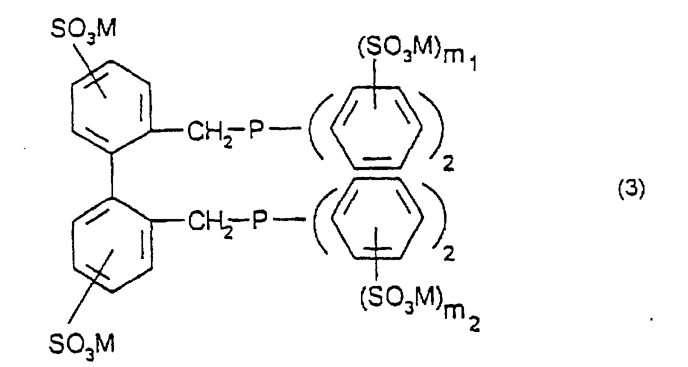 einsetzt, worin m₁ und m₂ unabhängig voneinander für 0 oder 1 steht und die Verbindungen der Formel (3) drei bis sechs -SO₃M Gruppen, worin M die vorstehend genannte Bedeutung besitzt, enthalten.

14. Verfahren nach einem oder mehreren der Ansprüche 1 bis 3, **dadurch gekennzeichnet, daß** man als sulfonierte Triarlyphosphine Gemische der Verbindungen der Formel (4) 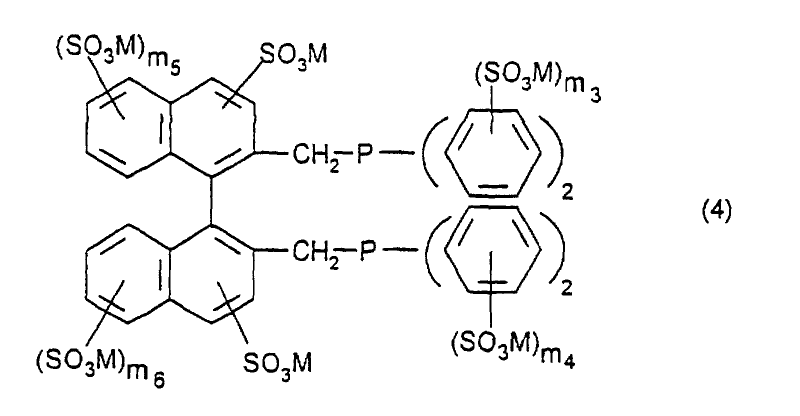 einsetzt, worin m₃, m₄, m₅ und m₆ unabhängig voneinander für 0 oder 1 steht und die Verbindungen der Formel (4) vier bis acht -SO₃M Gruppen, worin M die vorstehend genannten Bedeutungen besitzt, enthalten.

15. Verfahren nach einem oder mehreren der Ansprüche 13 und 14, **dadurch gekennzeichnet, daß** man Rhodium und sulfonierte Triarylphosphine der Formel (3) oder (4) im Molverhältnis 1 : 5 bis 1 : 100 einsetzt.

16. Verfahren nach einem oder mehreren der Ansprüche 13 bis 15, **dadurch gekennzeichnet, daß** man Rhodium und sulfonierte Triarylphosphine der Formel (3) und (4) im Molverhältnis 1 : 5 bis 1 : 50 einsetzt.

17. Verfahren nach einem oder mehreren der Ansprüche 13 bis 16, **dadurch gekennzeichnet, daß** man Rhodium und sulfonierte Triarylphosphine der Formel (3) oder (4) im Molverhältnis 1 : 8 bis 1 : 15 einsetzt.

18. Verfahren nach einem oder mehreren der Ansprüche 13 bis 17, **dadurch gekennzeichnet, daß** die wäßrige Phase bei Verwendung sulfonierter Triarylphosphine der Formel (3) oder (4) 20 bis 500 ppm Rhodium enthält.

19. Verfahren nach einem oder mehreren der Ansprüche 13 bis 18, **dadurch gekennzeichnet, daß** die wäßrige Phase bei Verwendung sulfonierter Triarylphosphine der Formel (3) oder (4) 30 bis 150 ppm Rhodium enthält.

20. Verfahren nach einem oder mehreren der Ansprüche 13 bis 19, **dadurch gekennzeichnet, daß** die wäßrige Phase bei Verwendung sulfonierter Triarylphosphine der Formel (3) oder (4) 40 bis 100 ppm Rhodium enthält.

21. Verfahren nach einem oder mehreren der Ansprüche 1 bis 20, **dadurch gekennzeichnet, daß** man ein aliphatisches α-Olefin oder cycloaliphatisches Olefin mit 6 bis 8 Kohlenstoffatomen in Anwesenheit einer wäßrigen Phase umsetzt, die mehr als 10 Gew.-% und bis zu 50 Gew.-% der Verbindung der Formel (1) enthält.

22. Verfahren nach einem oder mehreren der Ansprüche 1 bis 21, **dadurch gekennzeichnet, daß** man ein aliphatisches α-Olefin oder ein cycloaliphatisches Olefin mit 6 bis 8 Kohlenstoffatomen in Anwesenheit einer 20 bis 40 Gew.-% der Verbindung der Formel (1), enthaltenden wäßrigen Phase umsetzt.

23. Verfahren nach einem oder mehreren der Ansprüche 1 bis 20, **dadurch gekennzeichnet, daß** man ein aliphatisches α-Olefin oder cycloaliphatisches Olefin mit 9 bis 12 Kohlenstoffatomen in Anwesenheit einer 30 bis 70 Gew.-% der Verbindung der Formel (1) enthaltenden wäßrigen Phase, umsetzt.

24. Verfahren nach einem oder mehreren der Ansprüche 1 bis 20, **dadurch gekennzeichnet, daß** man ein aliphatisches α-Olefin oder cycloaliphatisches Olefin mit 9 bis 12 Kohlenstoffatomen in Anwesenheit einer 50 bis 60 Gew.-% der Verbindung der Formel (1) enthaltenden wäßrigen Phase, umsetzt.

25. Verfahren nach einem oder mehreren der Ansprüche 1 bis 24, **dadurch gekennzeichnet, daß** man ein Polyethylenglykol der Formel
H(OCH₂CH₂)ₙOH,
worin n für eine ganze Zahl von 3 bis 50 steht, als Verbindung der Formel (1) einsetzt.

26. Verfahren nach einem oder mehreren der Ansprüche 1 bis 25, **dadurch gekennzeichnet, daß** man ein Polyethylenglykol der Formel H(OCH₂CH₂)ₙOH, worin n für eine ganze Zahl von 4 bis 30 steht, als Verbindung der Formel (1) einsetzt.

27. Verfahren nach einem oder mehreren der Ansprüche 1 bis 26, **dadurch gekennzeichnet, daß** man eine Verbindung der Formel R(OCH₂CH₂)ₙOH, worin R für einen Methylrest oder β-Hydroxypropylrest und n für eine ganze Zahl von 3 bis 50 steht, als Verbindungen der Formel (1) einsetzt.

28. Verfahren nach einem oder mehreren der Ansprüche 1 bis 27, **dadurch gekennzeichnet, daß** man eine Verbindung der Formel R(OCH₂CH₂)ₙOH, worin R für einen Methylrest oder β-Hydroxypropylrest und n für eine ganze Zahl von 4 bis 30 steht, als Verbindungen der Formel (1) einsetzt.

29. Verfahren nach einem oder mehreren der Ansprüche 1 bis 28, **dadurch gekennzeichnet, daß** man die Umsetzung bei 50 bis 150 °C durchführt.

30. Verfahren nach einem oder mehreren der Ansprüche 1 bis 29, **dadurch gekennzeichnet, daß** man die Umsetzung bei 100 bis 140 °C durchführt.

31. Verfahren nach einem oder mehreren der Ansprüche 1 bis 30, **dadurch gekennzeichnet, daß** man die Umsetzung bei 20 bis 150 bar durchführt.

32. Verfahren nach einem oder mehreren der Ansprüche 1 bis 30, **dadurch gekennzeichnet, daß** man die Umsetzung bei 30 bis 80 bar durchführt.

## Claims

1. A process for preparing aldehydes reacting an olefinically unsaturated compound having from 6 to 16 carbon atoms with hydrogen and carbon monoxide in the presence of a solubilizer and of an aqueous phase comprising rhodium and sulfonated triarylphosphines as catalyst, which comprises reacting in the presence of an aqueous phase comprising more than 10 and up to 70% by weight of a mixture of compounds of the formula (1) R(OCH₂CH₂)ₙOR¹, where, in the formula (1), R is hydrogen, a straight-chain or branched alkyl radical having from 1 to 4 carbon atoms or a hydroxy alkyl radical having from 1 to 4 carbon atoms, R¹ is hydrogen or a methyl radical and n is an integer from 3 to 50, that the triarylphosphines have at least three -(SO₃)M groups, where M is identical or different and is H, an alkali metal ion, an ammonium ion, a quaternary ammonium ion, a ½ alkaline earth metal ion or ½ zinc ion, and that the reaction is carried out at from 20 bis 170°C and from 1 to 300 bar.

2. The process as claimed in claim 1, wherein the olefinically unsaturated compound used is an aliphatic olefin, cycloaliphatic olefin or araliphatic olefin having from 6 to 16 carbon atoms.

3. The process as claimed in claim 1 or 2, wherein the olefinic compound used is an aliphatic α-olefin having from 6 to 12 carbon atoms.

4. The process as claimed in one or more of claims 1 to 3, wherein the trisulfonated triarylphosphines used are compound of the formula (2) 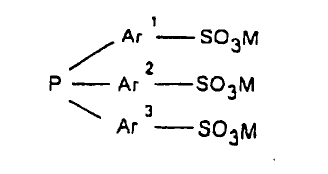 where Ar¹, Ar² and Ar³ are identical or different and are each a phenyl or naphthyl radical and M are identical or different and are each an alkali metal ion, an ammonium ion, a quaternary ammonium ion or a ½ alkaline earth metal ion or ½ zinc ion.

5. The process as claimed in one or more of claims 1 to 4, wherein the sulfonated triarylphosphine used is a sulfonated triphenylphosphine.

6. The process as claimed in one or more of claims 1 to 4, wherein the sulfonated triarylphosphine used is trisodium tri(m-sulfophenyl)phosphine.

7. The process as claimed in one or more of claims 1 to 6, wherein the aqueous phase is used in an amount corresponding to from 2 x 10⁻⁶ to 5 x 10⁻² mol of rhodium per mol of olefinic compound.

8. The process as claimed in one or more of claims 4 to 7, wherein rhodium and sulfonated triarylphosphines of the formula (2) are used in a molar ratio of from 1 : 10 to 1 : 1000.

9. The process as claimed in one or more of claims 4 to 8, wherein rhodium and sulfonated triarylphosphines of the formula (2) are used in a molar ratio of from 1 : 50 to 1 : 200.

10. The process as claimed in one or more of claims 4 to 9, wherein the aqueous phase contains from 100 to 1000 ppm of rhodium when using sulfonated triarylphosphines of the formula (2).

11. The process as claimed in one or more of claims 4 to 10, wherein the aqueous phase contains from 200 to 500 ppm of rhodium when using sulfonated triarylphosphines of the formula (2).

12. The process as claimed in one or more of claims 4 to 11, wherein the aqueous phase contains from 300 to 400 ppm of rhodium when using sulfonated triarylphosphines of the formula (2).

13. The process as claimed in one or more of claims 1 to 3, wherein the sulfonated triarylphosphines used are compounds of the formula (3) 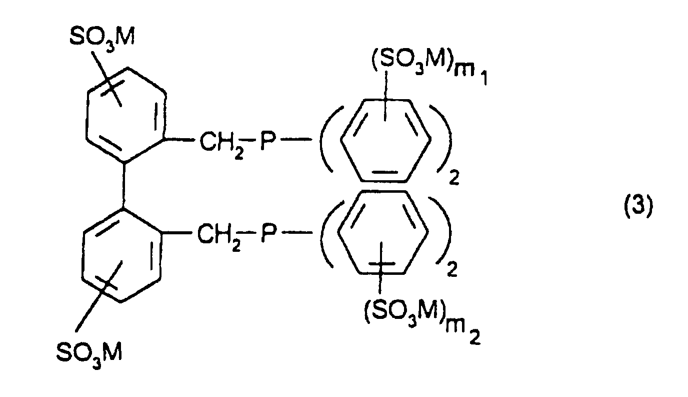 where m₁/and m₂ are, independently of one another, 0 or 1 and the compounds of the formula (3) contain from three to six -SO₃M groups, where M is as defined above.

14. The process as claimed in one or more of claims 1 to 3, wherein the sulfonated triarylphsophines used are compounds of the formula (4) 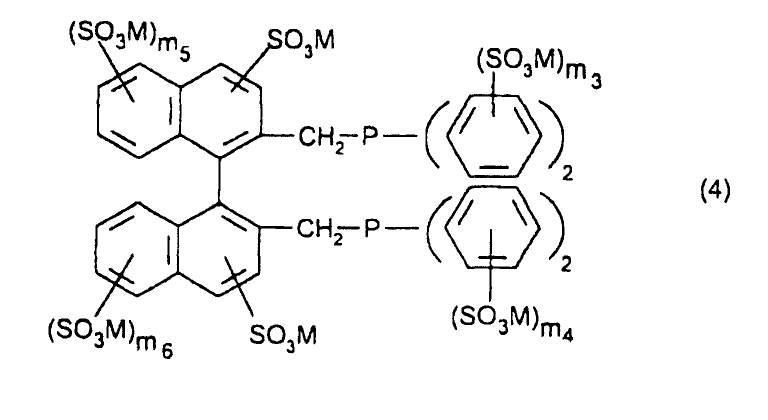 where m₃, m₄, m₅ and m₆ are, independently of one another, 0 or 1 and the compounds of the formula (4) have from four to eight -SO₃M groups, where m is as defined above.

15. The process as claimed in one or more of claims 13 and 14, wherein rhodium and sulfonated triarylphosphines of the formula (3) or (4) are used in a molar ratio of 1 : 5 to 1 : 100.

16. The process as claimed in one or more of claims 13 to 15, wherein rhodium and sulfonated triarylphosphines of the formula (3) or (4) are used in a molar ratio of 1 : 5 to 1 : 50.

17. The process as claimed in one or more of claims 13 to 16, wherein rhodium and sulfonated triarylphosphines of the formula (3) or (4) are used in a molar ratio of 1 : 8 to 1 : 15.

18. The process as claimed in one or more of claims 13 to 17, wherein the aqueous phase contains from 20 to 500 ppm of rhodium when using sulfonated triarylphosphines of the formula (3) or (4).

19. The process as claimed in one or more of claims 13 to 18, wherein the aqueous phase contains from 30 to 150 ppm of rhodium when using sulfonated triarylphosphines of the formula (3) or (4).

20. The process as claimed in one or more of claims 13 to 19, wherein the aqueous phase contains from 40 to 100 ppm of rhodium when using sulfonated triarylphosphines of the formula (3) or (4).

21. The process as claimed in one or more of claims 1 to 20, wherein an aliphatic α-olefin or cycloaliphatic olefin having from 6 to 8 carbon atoms is reacted in the presence of an aqueous phase containg more than 10 and up to 50% by weight of the compound of the formula (1).

22. The process as claimed in one or more of claims 1 to 21, wherein an aliphatic α-olefin or a cycloaliphatic olefin having from 6 to 8 carbon atoms is reacted in the presence of an aqueous phase containing from 20 to 40% by weight of the compound of the formula (1).

23. The process as claimed in one or more of claims 1 to 20, wherein an aliphatic α-olefin or cycloaliphatic olefin having from 9 to 12 carbon atoms is reacted in the presence of an aqueous phase containing from 30 to 70% by weight of the compound of the formula (1).

24. The process as claimed in one or more of claims 1 to 20, wherein an aliphatic α-olefin or cycloaliphatic olefin having from 9 to 12 carbon atoms is reacted in the presence of an aqueous phase containing from 50 to 60% by weight of the compound of the formula (1).

25. The process as claimed in one or more of claims 1 to 24, wherein the compound of the formula (1) used is a polyethylene glycol of the formula H(OCH₂CH₂)ₙOH, where n is an integer from 3 to 50.

26. The process as claimed in one or more of claims 1 to 25, wherein the compound of the formula (1) used is a polyethylene glycol of the formula H(OCH₂CH₂)ₙOH, where n is an integer from 4 to 30.

27. The process as claimed in one or more of claims 1 to 26, wherein the compound of the formula (1) used is a compound of the formula R(OCH₂CH₂)ₙOH, where R is a methyl radical or β-hydroxypropyl radical and n is an integer from 3 to 50.

28. The process as claimed in one or more of claims 1 to 27, wherein the compound of the formula (1) used is a compound of the formula R(OCH₂CH₂)ₙOH, where R is a methyl radical or β-hydroxypropyl radical and n is an integer from 4 to 30.

29. The process as claimed in one or more of claims 1 to 28, wherein the reaction is carried out at from 50 to 150°C.

30. The process as claimed in one or more of claims 1 to 29, wherein the reaction is carried out at from 100 to 140°C.

31. The process as claimed in one or more of claims 1 to 30, wherein the reaction is carried out at from 20 to 150 bar.

32. The process as claimed in one or more of claims 1 to 30, wherein the reaction is carried out at from 30 to 80 bar.

## Revendications

1. Procédé de préparation d'aldéhydes, selon lequel on fait réagir un composé à insaturation oléfinique de 6 à 16 atomes de carbone avec de l'hydrogène et du monoxyde de carbone en présence d'agents de solubilisation et d'une phase aqueuse contenant du rhodium et des triarylphosphines sulfonées comme catalyseur, **caractérisé en ce que** l'on travaille en présence d'une phase aqueuse contenant plus de 10 % en masse et jusqu'à 70 % en masse d'un mélange de composés de formule (1) R(OCH₂CH₂)ₙOR¹ où, dans la formule (1), R représente l'hydrogène, un reste alkyle linéaire ou ramifié de 1 à 4 atomes de carbone ou un reste hydroxyalkyle de 1 à 4 atomes de carbone, R¹ représente l'hydrogène ou un reste méthyle, et n représente un nombre entier de 3 à 50, **en ce que** les triarylphosphines contiennent au moins 3 restes -(SO₃)M, dans lesquels les M sont identiques ou différents et M représente H, un ion de métal alcalin, un ion ammonium, un ion ammonium quaternaire, 1/2 ion de métal alcalino-terreux ou 1/2 ion de zinc, et **en ce que** la réaction s'effectue à une température de 20 à 170°C et sous 1 à 300 bars.

2. Procédé selon la revendication 1, **caractérisé en ce que** l'on utilise comme composé à insaturation oléfinique une oléfine aliphatique, une oléfine cycloaliphatique ou une oléfine araliphatique de 6 à 16 atomes de carbone.

3. Procédé selon la revendication 1 ou 2, **caractérisé en ce que** l'on utilise comme composé oléfinique une α-oléfine aliphatique de 6 à 12 atomes de carbone.

4. Procédé selon l'une ou plusieurs des revendications 1 à 3, **caractérisé en ce que** l'on utilise comme triarylphosphines sulfonées des composés de formule (2) 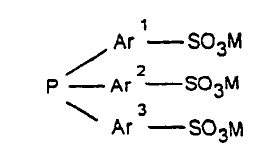 dans laquelle Ar¹, Ar² et Ar³ sont identiques ou différents et représentent chacun un reste phényle ou naphtyle et les M sont identiques ou différents et représentent un ion de métal alcalin, un ion ammonium, un ion ammonium quaternaire, 1/2 ion de métal alcalino-terreux ou 1/2 ion de zinc.

5. Procédé selon l'une ou plusieurs des revendications 1 à 4, **caractérisé en ce que** l'on utilise comme triarylphosphine sulfonée une triphénylphosphine sulfonée.

6. Procédé selon l'une ou plusieurs des revendications 1 à 4, **caractérisé en ce que** l'on utilise comme triarylphosphine sulfonée la tri(m-sulfophényl)phosphine trisodique.

7. Procédé selon l'une ou plusieurs des revendications 1 à 6, **caractérisé en ce que** l'on utilise une phase aqueuse correspondant à une quantité de 2 × 10⁻⁶ à 5 × 10⁻² mol de rhodium par mol de composé oléfinique.

8. Procédé selon l'une ou plusieurs des revendications 4 à 7, **caractérisé en ce que** l'on utilise le rhodium et les triarylphosphines sulfonées de formule (2) en un rapport molaire de 1:10 à 1:1000.

9. Procédé selon l'une ou plusieurs des revendications 4 à 8, **caractérisé en ce que** l'on utilise le rhodium et les triarylphosphines sulfonées de formule (2) en un rapport molaire de 1:50 à 1:200.

10. Procédé selon l'une ou plusieurs des revendications 4 à 9, **caractérisé en ce que** la phase aqueuse contient 100 à 1000 ppm de rhodium dans le cas de l'utilisation de triarylphosphines sulfonées de formule (2).

11. Procédé selon l'une ou plusieurs des revendications 4 à 10, **caractérisé en ce que** la phase aqueuse contient 200 à 500 ppm de rhodium dans le cas de l'utilisation de triarylphosphines sulfonées de formule (2).

12. Procédé selon l'une ou plusieurs des revendications 4 à 11, **caractérisé en ce que** la phase aqueuse contient 300 à 400 ppm de rhodium dans le cas de l'utilisation de triarylphosphines sulfonées de formule (2).

13. Procédé selon l'une ou plusieurs des revendications 1 à 3, **caractérisé en ce que** l'on utilise comme triarylphosphines sulfonées des mélanges de composés de formule (3) 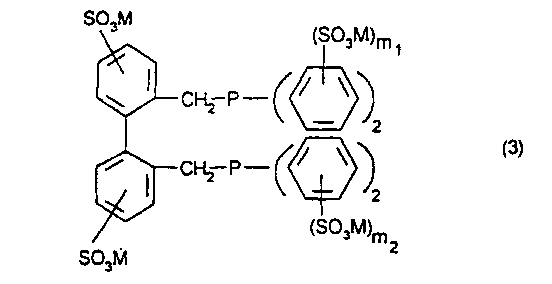 dans laquelle les m₁ et m₂ représentent, indépendamment les uns des autres, 0 ou 1, et les composés de formule (3) contiennent 3 à 6 groupes -SO₃M, M ayant la signification indiquée précédemment.

14. Procédé selon l'une ou plusieurs des revendications 1 à 3, **caractérisé en ce que** l'on utilise comme triarylphosphines sulfonées des mélanges de composés de formule (4) 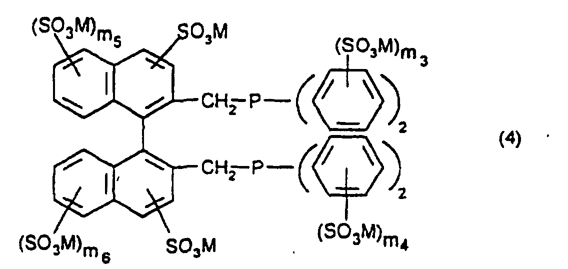 dans laquelle m₃, m₄, m₅ et m₆ représentent indépendamment les uns des autres 0 ou 1 et les composés de formule (4) contiennent 4 à 8 groupes -SO₃M, M ayant la signification indiquée précédemment.

15. Procédé selon l'une ou plusieurs des revendications 13 et 14, **caractérisé en ce que** l'on utilise le rhodium et les triarylphosphines sulfonées de formule (3) ou (4) en un rapport molaire de 1:5 à 1:100.

16. Procédé selon l'une ou plusieurs des revendications 13 à 15, **caractérisé en ce que** l'on utilise le rhodium et les triarylphosphines sulfonées de formule (3) ou (4) en un rapport molaire de 1:5 à 1:50.

17. Procédé selon l'une ou plusieurs des revendications 13 à 16, **caractérisé en ce que** l'on utilise le rhodium et les triarylphosphines sulfonées de formule (3) ou (4) en un rapport molaire de 1:8 à 1:15.

18. Procédé selon l'une ou plusieurs des revendications 13 à 17, **caractérisé en ce que** la phase aqueuse contient 20 à 500 ppm de rhodium dans le cas de l'utilisation de triarylphosphines sulfonées de formule (3) ou (4).

19. Procédé selon l'une ou plusieurs des revendications 13 à 18, **caractérisé en ce que** la phase aqueuse contient 30 à 150 ppm de rhodium dans le cas de l'utilisation de triarylphosphines sulfonées de formule (3) ou (4).

20. Procédé selon l'une ou plusieurs des revendications 13 à 19, **caractérisé en ce que** la phase aqueuse contient 40 à 100 ppm de rhodium dans le cas de l'utilisation de triarylphosphines sulfonées de formule (3) ou (4).

21. Procédé selon l'une ou plusieurs des revendications 1 à 20, **caractérisé en ce que** l'on fait réagir une α-oléfine aliphatique ou une oléfine cycloaliphatique de 6 à 8 atomes de carbone en présence d'une phase aqueuse qui contient plus de 10 % en masse et jusqu'à 50 % en masse du composé de formule (1).

22. Procédé selon l'une ou plusieurs des revendications 1 à 21, **caractérisé en ce que** l'on fait réagir une α-oléfine aliphatique ou une oléfine cycloaliphatique de 6 à 8 atomes de carbone en présence d'une phase aqueuse contenant 20 à 40 % en masse du composé de formule (1).

23. Procédé selon l'une ou plusieurs des revendications 1 à 20, **caractérisé en ce que** l'on fait réagir une α-oléfine aliphatique ou une oléfine cycloaliphatique de 9 à 12 atomes de carbone en présence d'une phase aqueuse contenant 30 à 70 % en masse du composé de formule (1).

24. Procédé selon l'une ou plusieurs des revendications 1 à 20, **caractérisé en ce que** l'on fait réagir une α-oléfine aliphatique ou une oléfine cycloaliphatique de 9 à 12 atomes de carbone en présence d'une phase aqueuse contenant 50 à 60 % en masse du composé de formule (1).

25. Procédé selon l'une ou plusieurs des revendications 1 à 24, **caractérisé en ce que** l'on utilise comme composé de formule (1) un polyéthylèneglycol de formule H(OCH₂CH₂)ₙOH dans laquelle n représente un nombre entier de 3 à 50.

26. Procédé selon l'une ou plusieurs des revendications 1 à 25, **caractérisé en ce que** l'on utilise comme composé de formule (1) un polyéthylèneglycol de formule H(OCH₂CH₂)ₙOH dans laquelle n représente un nombre entier de 4 à 30.

27. Procédé selon l'une ou plusieurs des revendications 1 à 26, **caractérisé en ce que** l'on utilise comme composé de formule (1) un composé de formule R(OCH₂CH₂)ₙOH dans laquelle R représente un reste méthyle ou un reste β-hydroxypropyle et n représente un nombre entier de 3 à 50.

28. Procédé selon l'une ou plusieurs des revendications 1 à 27, **caractérisé en ce que** l'on utilise comme composé de formule (1) un composé de formule R(OCH₂CH₂)ₙOH dans laquelle R représente un reste méthyle ou un reste β-hydroxypropyle et n représente un nombre entier de 4 à 30.

29. Procédé selon l'une ou plusieurs des revendications 1 à 28, **caractérisé en ce que** l'on effectue la réaction à une température de 50 à 150°C.

30. Procédé selon l'une ou plusieurs des revendications 1 à 29, **caractérisé en ce que** l'on effectue la réaction à une température de 100 à 140°C.

31. Procédé selon l'une ou plusieurs des revendications 1 à 30, **caractérisé en ce que** l'on effectue la réaction sous une pression de 20 à 150 bars.

32. Procédé selon l'une ou plusieurs des revendications 1 à 30, **caractérisé en ce que** l'on effectue la réaction sous une pression de 30 à 80 bars.
